(19) Europäisches Patentamt
European Patent Office
Office européen des brevets

(11) EP 0 958 288 B1

# (12) EUROPÄISCHE PATENTSCHRIFT

(45) Veröffentlichungstag und Bekanntmachung des Hinweises auf die Patenterteilung:
**03.03.2004 Patentblatt 2004/10**

(21) Anmeldenummer: **98956789.6**

(22) Anmeldetag: **22.09.1998**

(51) Int Cl.[7]: **C07D 311/92**, C07D 311/78, C08K 5/15, G02B 5/23

(86) Internationale Anmeldenummer:
**PCT/DE1998/002820**

(87) Internationale Veröffentlichungsnummer:
**WO 1999/015518 (01.04.1999 Gazette 1999/13)**

(54) **PHOTOCHROME NAPHTHOPYRAN-FARBSTOFFE, VERFAHREN ZU IHRER HERSTELLUNG, IHRE VERWENDUNG SOWIE EIN PHOTOCHROMER GEGENSTAND**

PHOTOCHROMIC NAPHTOPYRANE COLORANTS, METHOD FOR THE PRODUCTION AND USE THEREOF, PHOTOCHROMIC OBJECT

COLORANTS DE NAPHTOPYRANE PHOTOCHROMIQUES, LEUR PROCEDE DE PRODUCTION, LEUR UTILISATION, AINSI QU'OBJET PHOTOCHROMIQUE

(84) Benannte Vertragsstaaten:
**DE FR GB IT**

(30) Priorität: **22.09.1997 DE 19741705**
**27.04.1998 DE 19818684**

(43) Veröffentlichungstag der Anmeldung:
**24.11.1999 Patentblatt 1999/47**

(73) Patentinhaber: **Rodenstock GmbH**
**80469 München (DE)**

(72) Erfinder:
- **MELZIG, Manfred**
  **D-82234 Wessling (DE)**
- **MANN, Claudia**
  **D-80634 München (DE)**
- **WEIGAND, Udo**
  **D-80638 München (DE)**

(74) Vertreter: **Meissner, Bolte & Partner**
**Anwaltssozietät GbR**
**Postfach 86 06 24**
**81633 München (DE)**

(56) Entgegenhaltungen:
**WO-A-96/14596**
**WO-A-97/21698**
**WO-A-97/48762**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung bezieht sich auf photochrome Verbindungen, nämlich indenoannellierte Naphthopyran-Farbstoffe mit unterschiedlichen Substituenten, ein Verfahren zu ihrer Herstellung, ihre Verwendung sowie einen photochromen Gegenstand, der die photochromen Naphthopyran-Farbstoffe umfaßt.

Es sind verschiedene Farbstoffklassen bekannt, die bei Bestrahlung mit Licht bestimmter Wellenlängen, insbesondere Sonnenlicht, reversibel ihre Farbe wechseln. Dies rührt daher, daß diese Farbstoffmoleküle durch Energiezufuhr in Form von Licht in einen angeregten Zustand übergehen, den sie bei Unterbrechung der Energiezufuhr wieder verlassen und in ihren Ausgangszustand zurückkehren. Zu diesen photochromen Farbstoffen gehören beispielsweise die Naphthopyrane, die im Stand der Technik mit verschiedenen Substituenten bereits beschrieben wurden.

Stand der Technik

Pyrane, speziell Naphthopyrane und von diesen abgeleitete größere Ringsysteme, sind die derzeit am meisten bearbeitete Klasse photochromer Verbindungen. Obwohl bereits im Jahr 1966 erstmals zum Patent angemeldet (US 3.567.605), konnten erst in den 90er Jahren Verbindungen entwickelt werden, die für den Einsatz in Brillengläsern geeignet erschienen. Eine einsetzbare Verbindungsklasse von Pyranen sind zum Beispiel die 2,2-Diaryl-2H-naphtho [1,2-b]pyrane, die in angeregter Form verschiedene Färbungen, wie gelb, orange oder rotorange, zeigen. Als weitere Verbindungsklasse photochromer Verbindungen sind die indenoannellierten Naphthopyrane von Interesse, die aufgrund ihres größeren Ringsystems längerwellig absorbieren. Es sind von den 2,2-Diaryl-2H-naphtho[1,2-b]pyranen abgeleitete Systeme, die durch Annellierung an der f-Seite aus den jeweiligen Naphthopyransystemen hervorgehen.

Beispielsweise beschreiben die WO 96/14596 bzw. die US 5.645.767 Indeno [2,1-f]naphtho[1,2-b]pyran-Systeme, diese photochromen Farbstoffe enthaltende Zusammensetzungen, sowie ein Verfahren zur Herstellung dieser Verbindungen. Die in diesen Druckschriften beschriebenen Naphthopyran-Systeme sollen dahingehend modifiziert werden, daß sie eine schnellere Aufhellung als bisher bekannte 2H-Naphthopyrane erzielen. Zur Lösung sind beispielhaft als Reste R in Position 13 nur Wasserstoff und Alkylgruppen beschrieben.

Die in der WO 96/14596 für B und B' beanspruchte, sehr umfangreiche Substituentenliste an den Aryl- oder Heteroarylgruppen führt neben Amino, Monound Di-($C_1$-$C_6$)-Alkylaminogruppen, Piperidin, Morpholin und Pyrrol auf, wobei hierzu Einzelheiten fehlen.

Die oben beschriebenen Verbindungen können jedoch Fulvene bilden, die bei $\alpha$-Wasserstoff tragenden Alkylgruppen unter Wasserabspaltung entstehen. Diese tieffarbigen Zerstörungsprodukte werden u.a. beim Lebensdauertest (Xenonlicht) gebildet. Darüber hinaus können diese photochromen Farbstoffe mit den in der WO 96/14596 bzw. der US 5.645.767 beanspruchten Substituenten nur den kürzerwelligen Teil des Spektrums abdecken, wobei der Farbeindruck orange bis blau/grau ist (siehe zum Beispiel Beschreibung der WO 96/14596, S. 2, Z. 24/25).

Ferner werden im Herstellungsverfahren nach diesen Druckschriften zum Teil sehr aggressive Reaktionsbedingungen eingesetzt, beispielsweise erfolgt ein einstündiges Erhitzen mit konzentrierter Phosphorsäure bei Temperaturen von 160 bis 220°C (vergleiche Reaktion (5): Cyclisierung von D zu E). Dadurch sind Substituenten mit empfindlicheren Gruppierungen nicht einsetzbar, da diese unter derartigen Bedingungen oxidiert oder abgebaut werden würden. Auf dem in der WO 96/14596 bzw. der US 5.645.767 beschriebenen Syntheseweg, nämlich Schließung des 6-Rings vor dem 5-Ring, sind darüber hinaus bestimmte Verbindungen überhaupt nicht oder nur schwer darstellbar.

Zudem sind die oben beschriebenen Verbindungen mit Nachteilen verbunden, da die im Handel erhältlichen Gläser mit Verbindungen aus dem Stand der Technik den Brillenträger beeinträchtigen, weil unter bestimmten Bedingungen starke Farbverfälschungen auftreten. Bei großer Kälte und intensiver UV-Bestrahlung, wie dies zum Beispiel beim Gletscher-Skilaufen der Fall ist, wird im Bereich 380-620 nm mindestens 90% des einfallenden Lichts absorbiert. Da die Absorption zu größeren Wellenlängen hin (roter Spektralbereich) steil abfällt, sieht der Brillenträger die Umgebung stark rotbetont (sogenannter "blutroter Schnee").

Hintergrund der Erfindung

Der Erfindung liegt daher die Aufgabe zugrunde, die beschriebenen Nachteile des Standes der Technik zu vermeiden und photochrome Verbindungen bereitzustellen, die verbesserte Eigenschaften gegenüber den im Stand der Technik beschriebenen Strukturen besitzen und in der lichtangeregten Form gegenüber vergleichbaren Verbindungen aus dem Stand der Technik eine bathochrom verschobene längstwellige Absorption im sichtbaren Teil des Spektrums aufweisen. Demnach sollten Verbindungen gefunden werden, deren Absorptionsmaximum im Sichtbaren gegenüber den bekannten Verbindungen nochmals rund 100 nm bathochrom verschoben ist. Zusätzlich sollte die längstwellige Absorptionsbande, d.h. die Absorptionsbande zwischen dem roten bzw. infraroten Teil des Lichtspektrums und dem "Absorptionsloch" - der Stelle geringster Absorption im Bereich 400-500 nm - möglichst breit sein. Ein weiteres Ziel waren daher Verbindungen mit einem Absorptionsverhalten, das bei tiefer Eindunkelung (Transmission

bei $\lambda_{max}$ < 10%) bei 700 nm noch mindestens 50% und bei 750 nm noch mindestens 10% Absorption bietet.

Darüber hinaus sollten die bevorzugten Verbindungen keine ausgeprägte basische Funktion besitzen, da diese erfahrungsgemäß sowohl bei der Herstellung von photochromen Kunststoffgläsem im Massefärbungsverfahren leicht Protonen addieren und Farbabweichungen ergeben als auch im Dauergebrauch unter Lichteinwirkung zu Nebenreaktionen neigen, die zu einem Verlust der photochromen Reaktion führen.

Ferner sollte das Verfahren zur Herstellung der neuen Verbindungen gegenüber herkömmlichen Verfahren verbessert werden, wobei einfache Verfahrensführung, schonende Reaktionsbedingungen und gute Ausbeuten im Vordergrund stehen sollten.

Die oben erläuterte Aufgabe der Erfindung wird gelöst durch die photochromen Naphthopyran-Farbstoffe mit der allgemeinen Strukturformel (I)

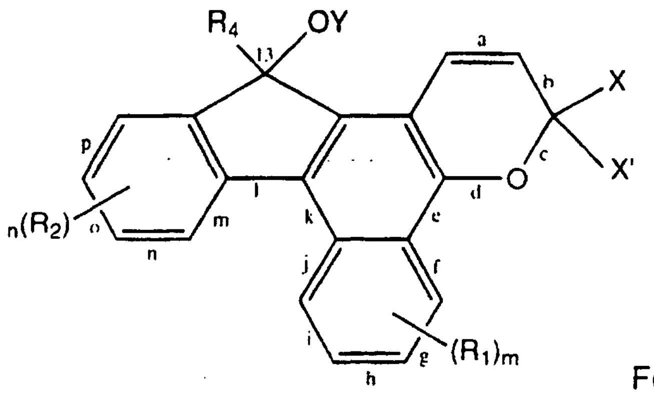

Formel (I)

wobei

$R_1$ und $R_2$ unabhängig voneinander gleich oder verschieden sind und ausgewählt sind aus

der Gruppe f, bestehend aus ($C_1$-$C_6$)-Alkyl, ($C_1$-$C_6$)-Alkoxy, Chloro und Fluoro;

oder

der Gruppe, bestehend aus Wasserstoff, ($C_5$-$C_6$)-Cycloalkyl, Phenyl, Benzyl, Dialkyl-($C_1$-$C_6$)-amino, Dicyclohexylamino, Diphenylamino, Piperidyl, Morpholinyl und Pyridyl, wobei m, n = 0, 1 oder 2 sind;

oder

zwei $R_1$ und/oder zwei $R_2$ bilden zusammen, unabhängig voneinander, einen carbo- oder heterocyclischen Ring, ausgewählt aus Benzol, Pyridin, Pyrazin, Pyrimidin, Furan und Thiophen;

$R_4$ ausgewählt ist aus

der Gruppe g, bestehend aus ($C_5$-$C_6$)-Cycloalkyl, ($C_1$-$C_6$)-Acyl, ($C_1$-$C_6$)-Alkoxy, Phenyl, Benzyl, monosubstituiertem Phenyl, monosubstituiertem Benzyl, wobei die Arylsubstituenten ($C_1$-$C_6$)-Alkyl oder ($C_1$-$C_6$)-Alkoxy sind;

oder aus

monochlor- oder monofloursubstituiertes Phenyl, Naphtyl, Phenanthryl, Pyrenyl, Chinolyl, Isochinolyl, Benzofuranyl, Benzothienyl, Dibenzofuranyl, Dibenzothienyl, Carbazolyl, oder Indolyl, jeweils unsubstituiert oder monosubstituiert, wobei die Substituenten ausgewählt sind aus der Gruppe bestehend aus Chloro, Fluoro, ($C_1$-$C_6$)-Alkyl oder ($C_1$-$C_6$)-Alkoxy;

oder aus

di- oder trisubstituierten Phenyl ausgewählt sind aus der Gruppe bestehend aus Chloro, Fluoro, ($C_1$-$C_6$)-Alkyl, ($C_1$-$C_6$)-Alkoxy, ($C_1$-$C_6$)-ω-Phenylalkyl und ($C_1$-$C_6$)-ω-Phenoxyalkyl, wobei der ω-ständige Phenylring kann dabei wiederum den X, X' oder $R_4$-Rest eines weiteren photochromen Pyransystems darstellen kann;

X und X' unabhängig voneinander ausgewählt sind aus der Gruppe z, bestehend aus a) und b) mit

a) den un-, mono-, di- und trisubstituierten Arylgruppen Phenyl und Naphthyl und

b) den un-, mono-, di- und trisubstituierten heterocyclischen Gruppen Pyridyl, Furanyl, Benzofuran-2-yl,

Benzofuran-3-yl, Thienyl, Benzothien-2-yl und Benzothien-3-yl,

und die Substituenten in a) und b) ausgewählt sind aus

der Gruppe h, bestehend aus Hydroxy, Amino, Mono-($C_1$-$C_6$)-alkylamino, Di-($C_1$-$C_6$)-alkylamino, Piperidino, Morpholino, Pyrryl, ($C_1$-$C_6$)-Alkyl, ($C_1$-$C_6$)-Chloralkyl, ($C_1$-$C_6$)-Fluoralkyl, ($C_1$-$C_6$)-Alkoxy, Mono-($C_1$-$C_6$)-alkoxy-($C_1$-$C_4$)-alkyl, Chloro und Fluoro; oder

der Gruppe a, bestehend aus nichtbasischen Aminen, wie unsubstituiertem oder substituiertem Diarylamino, Pyrazol, Imidazol, Indol, Pyridin, Pyrazolin, Imidazolin, Pyrrolin, Phenothiazin, Phenoxazin, Phenazin, Acridin oder Carbazol, wobei die Substituenten an den nichtbasischen Aminen ausgewählt sind aus ($C_1$-$C_4$)-Alkyl, ($C_1$-$C_4$)-Alkoxy, Phenyl, Fluoro, Chloro und Bromo;

oder ausgewählt sind aus

c) ($C_1$-$C_6$)-Alkyl, ($C_1$-$C_6$)-Chloralkyl, ($C_1$-$C_6$)-Fluoralkyl, Mono-($C_1$-$C_6$)-alkoxy-($C_1$-$C_4$)-alkyl, ($C_3$-$C_6$)-Cycloalkyl, Mono-($C_1$-$C_6$)-alkoxy-($C_3$-$C_6$)-cycloalkyl, Chlor-($C_3$-$C_6$)-cycloalkyl und Fluor-($C_3$-$C_6$)-cycloatkyl; oder

X und X' bilden mit dem Kohlenstoffatom der Verknüpfungsstelle mit dem Pyran einen gesättigten $C_5$-$C_{10}$ monocyclischen, $C_7$-$C_{12}$ bicyclischen oder $C_7$-$C_{12}$ tricyclischen Kohlenwasserstoffring oder ein Fluoren-9-yliden;

oder

entweder X oder X' ist mono- oder disubstituiertes Phenyl, das in para-Stellung zur Verknüpfung mit -Phenyl, -$(CH_2)_p$-Phenyl oder -O-$(CH_2)_p$-O-Phenyl substituiert ist (p = 1, 2, 3...,6), wobei der zweite Phenylring zu einem zweiten - nicht notwendigerweise identischen - photochromen Pyran gehört;

und

Y ausgewählt ist aus der Gruppe, bestehend aus Wasserstoff, ($C_1$-$C_6$)-Alkyl, ($C_1$-$C_6$)-Acyl, unsubstituiertem Benzoyl, unsubstituiertem Naphthoyl, monosubstituiertem Benzoyl und monosubstituiertem Naphthoyl, wobei die Arylsubstituenten ($C_1$-$C_6$)-Alkyl oder ($C_1$-$C_6$)-Alkoxy sind;

mit der Maßgabe, daß $R_1$ und $R_2$ nicht aus der Gruppe f sind, wenn $R_4$ aus der Gruppe g und X und X' aus der Gruppe z sind, für den Fall, daß die Reste der Gruppe z unsubstituiert oder die Substituenten aus der Gruppe h sind.

Überschneidungen zum nächsten Stand der Technik (WO 96/14596 bzw. US 5.645.767) bezüglich der Substituenten sind damit durch einen Disclaimer ausgeschlossen.

Nach einer erfindungsgemäßen Variante können die Heterocyclen in der Gruppe $R_4$ in den photochromen Naphthopyran-Farbstoffen über den Carbocyclus mit dem Naphthopyransystem verbunden sein, was jedoch nicht zwingend notwendig ist, um die mit der Erfindung erzielbaren Eigenschaften zu erreichen.

Nach einer erfindungsgemäßen Ausführungsform sind photochrome indenoannellierte Naphthopyrane mit den oben gezeigten allgemeinen chemischen Strukturformeln bevorzugt, wobei $R_1$, $R_2$, $R_4$ und Y wie oben definiert sind und X und X' ausgewählt sind aus der oben definierten Gruppe z, mit der Maßgabe, daß $R_1$ und $R_2$ nicht aus der Gruppe f sind, wenn $R_4$ aus der Gruppe g und X und X' aus der Gruppe z sind, für den Fall, daß die Reste der Gruppe z unsubstituiert oder die Substituenten aus der Gruppe h sind.

Erfindungsgemäß kann $R_4$ vorzugsweise auch aus der Gruppe, bestehend aus zumindest in ortho-Position substituierten Phenylresten, 1- oder 2-Naphthyl, 9-Phenanthryl, Pyrenyl und Heteroaromaten, wie Benzofuranyl, Benzothienyl, Chinolyl oder Isochinolyl ausgewählt sein.

In den photochromen Naphthopyran-Farbstoffen ist es bevorzugt, daß zwei $R_1$ und/oder zwei $R_2$ unabhängig voneinander einen carbo- oder heterocyclischen Ring, ausgewählt aus Benzol, Pyridin, Pyrazin, Pyrimidin, Furan und Thiophen bilden.

Nach einer weiteren Ausführungsform der Erfindung können in den Naphthopyran-Farbstoffen mindestens ein X oder X' einen zumindest mit einem nichtbasischen Amin aus der Gruppe a substituierten oben definierten Rest aus der Gruppe z darstellen. X und X' können die gleichen Reste aus der Gruppe z mit unterschiedlichen Substituenten, beispielsweise aus der Gruppe a, darstellen. Alternativ können die Substituenten an X und X' beide gleich sein. Es ist dann von großem Vorteil, daß die erfindungsgemäßen Verbindungen keine basische Funktion im Molekül besitzen, d. h. eine sehr geringe oder keine Basizität zeigen. Die erfindungsgemäßen nichtbasischen Aminogruppen umfassen unsubstituiertes oder substituiertes Diarylamino, Pyrazol, Imidazol, Indol, Pyridin, Pyrazolin, Imidazolin, Pyrrolin, Phenothiazin, Phenoxazin, Phenazin, Acridin, Carbazol oder dergleichen.

Die nichtbasischen Aminosubstituenten der Gruppe a können generell unsubstituiert oder monosubstituiert vorliegen, wobei die Substituenten aus der Gruppe, bestehend aus ($C_1$-$C_4$)-Alkyl, ($C_1$-$C_4$)-Alkoxy, Phenyl, Fluoro, Chloro und Bromo, ausgewählt sein können. Dem Fachmann ist im Rahmen der Erfindung klar, daß jegliche Amine zur

Gruppe a gehören, ob diese offene, d.h. aliphatische Amine, oder auch cyclische Amine sind, solange sie keine ausgeprägt basische Funktion besitzen.

Bei Auswahl von nichtbasischen Aminosubstituenten werden im Gegensatz zu basischen Aminosubstituenten nachteilige Reaktionen bei der Verarbeitung der erfindungsgemäßen Farbstoffe und auch beim Langzeiteinsatz unter Lichteinwirkung generell ausgeschlossen. Die Lebensdauer der photochromen Farbstoffe wird somit wesentlich verlängert, es werden keine Protonen addiert, unerwünschte Farbabweichungen aufgrund von Nebenreaktionen erfolgen nicht. Basische Aminosubstituenten führen zudem häufig zu einer Beeinträchtigung der Oberflächenbeschaffenheit des Glases bei dessen Herstellung mittels Massefärbung, d.h. es treten aufgrund verminderter Glashaftung sog. Abplatzer auf. Demgegenüber wird bei Vorhandensein nichtbasischer Aminosubstituenten gemäß der vorliegenden Erfindung die Glashaftung bei der Herstellung massegefärbter Gläser nicht vermindert, so daß erheblich weniger Ausschuß in Form derartiger Abplatzer entsteht.

In überraschender Weise hat sich gezeigt, daß die Eigenschaften der erfindungsgemäßen Naphthopyran-Farbstoffe, wie Absorption, Migration und Aufhellgeschwindigkeit durch die Auswahl der Substituenten gezielt eingestellt werden können. Auch das Molekulargewicht des Farbstoffs kann durch Auswahl der Substituenten eingestellt und/oder vervielfacht werden.

Zusätzlich können in den erfindungsgemäßen indenoannellierten Naphthopyranen eine oder mehrere Methoxygruppen in 5-, 6-, 7- und/oder 8-Position des Naphthopyranrings, d.h. am Naphthalinring, vorhanden sein. Dies ist eine bathochrom wirkende Gruppierung, die sich additiv auf die bereits vorhandenen bathochrom wirkenden Gruppen auswirkt.

Überraschende und unerwartete Eigenschaften zeigen sich insbesondere bei Einführung von Diphenylaminogruppen anstelle von beispielsweise Methoxygruppen bei den indenoannellierten 2,2-Diaryl-2H-naphtho[1,2-b]-pyranen:

Bei den unsubstituierten 3,3-Diaryl-3H-naphtho[2,1-b]pyranen, wie sie aus der P 198 20 781.6 bekannt sind, bewirkt der Ersatz einer 4-Methoxygruppe an einem der Phenylringe in 3-Stellung des Naphthopyrans durch eine Diphenylaminogruppe eine bathochrome Verschiebung der längstwelligen Absorptionsbande um ca. 60 nm.

Beim Ersatz von 4-Methoxygruppen durch eine Diphenylaminogruppe an den Phenylringen in 2-Stellung der indenoannellierten 2,2-Diaryl-2H-naphtho[1,2-b]-pyranen werden daher grüne Verbindungen mit einem Absorptionsmaximum um 630 nm erwartet. Die Substitution bewirkt dagegen nur eine bathochrome Verschiebung um knapp 30 nm. Dafür wird die Absorptionsbande wesentlich breiter: sie erfüllt die gestellten Absorptionsbedingungen im roten reich des Sichtbaren. Eine zweite Absorption um 440 nm wird um ca. 60 nm bathochrom verschoben und bildet nunmehr eine Schulter zur Hauptbande.

Weitere besonders bevorzugte photochrome Naphthopyran-Farbstoffe besitzen die nachfolgende chemische Strukturformel (II):

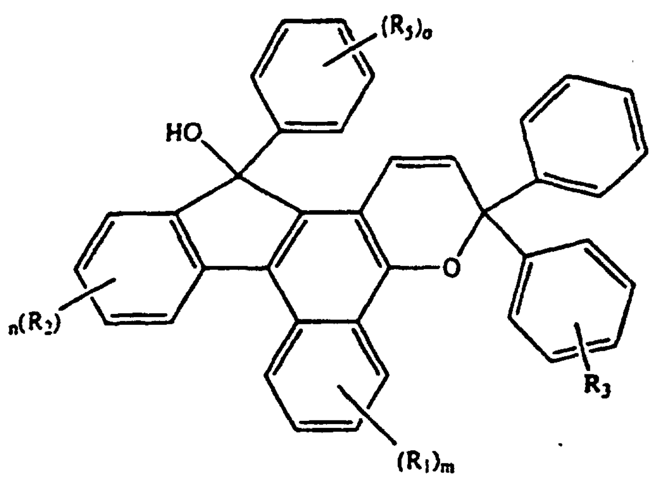

Formel (II)

wobei $R_1$, $R_2$, m und n wie oben definiert sind, $R_5$ die für $R_4$ = Phenyl definierten Substituenten mit o = 1, 2 oder 3 darstellt und $R_3$ ein nichtbasisches Amin aus der oben definierten Gruppe a ist.

Bevorzugt in Formel (II) ist der nichtbasische Aminosubstituent unsubstituiertes oder substituiertes Diarylamino, wobei zumindest ein Arylring mono-, di- oder trisubstituiert sein kann. Als Substituenten kommen hierfür $(C_1-C_4)$ -Alkyl, $(C_1-C_4)$-Alkoxy, Phenyl, Fluoro, Chloro oder Bromo in Frage.

Bevorzugte photochrome Naphthopyran-Farbstoffe der Erfindung sind:

1) 6-Methoxy-3-(4-methoxyphenyl)-3,13-diphenyl-13-hydroxy-benz[p]-indeno[2,1-f]naphtho[1,2-b]pyran,
2) 6-Methoxy-3-(4-methoxyphenyl)-3-phenyl-13-(2,5-dimethylphenyl)-13-hydroxy-benz[p]indeno[2,1-f]naphtho[1,2-b]pyran,
3) 3-(4-Diphenylaminophenyl)-3-phenyl-13-(2,5-dimethylphenyl)-13-hydroxybenz[p]indeno[2,1-f]naphtho[1,2-b]pyran,
4) 3-(4-Diphenylaminophenyl)-6-methoxy-3,13-diphenyl-13-hydroxyindeno[2,1-f]naphtho-[1,2-b]pyran,
5) 3-(4-Diphenylaminophenyl)-6-methoxy-3-phenyl-13-(2,5-dimethylphenyl)-13-hydroxy-indeno[2,1-f]naphtho[1,2-b]pyran,
6) 6-Methoxy-3-(4-methoxyphenyl)-3-phenyl-13-(2,5-dimethylphenyl)-13-hydroxy-indeno[2,1-f]naphtho[1,2-b]pyran,
7) 6-Methoxy-3-(4-methoxyphenyl)-3-phenyl-13-(1-naphthyl)-13-hydroxyindeno[2,1-f]-naphtho[1,2-b]pyran,
8) 6,11-Dimethoxy-3-(4-methoxyphenyl)-3-phenyl-13-(2,5-dimethylphenyl)-13-hydroxy-indeno[2,1-f]naphtho[1,2-b]pyran,
9) 3-(4-Methoxyphenyl)-3-phenyl-13-(2,5-dimethylphenyl)-13-hydroxyindeno[2,1-f]-naphtho[1,2-b]pyran und
10) 6-Methoxy-3-(4-methoxyphenyl)-3-phenyl-13-(3-methyl-2-thienyl)-13-hydroxy-indeno[2,1-f]naphtho[1,2-b]pyran.

Gegenstand der Erfindung ist auch ein Verfahren zur Herstellung der erfindungsgemäßen photochromen Naphthopyran-Farbstoffe. Im folgenden wird anhand der Figuren 1 und 2 der Syntheseweg im einzelnen erläutert, wobei auf die dort verwendeten Bezeichnungen Bezug genommen wird.

Die als Ausgangsmaterial verwendeten substituierten oder unsubstituierten Benzo- bzw. Naphthophenone A sind entweder im Handel erhältlich oder können durch Friedel-Crafts-Acylierung gemäß Schritt (1) leicht und meist in guten Ausbeuten erhalten werden.

In Schritt (2) wird über eine Stobbe-Kondensation mittels Bernsteinsäurediester der Halbester B erzeugt. Sofern die beiden über die Ketogruppe verbundenen Ringe nicht identisch sind, entsteht ein Isomerengemisch. Abweichend von der Darstellung in der WO 96/14596 wird erfindungsgemäß mit dem Isomerengemisch weitergearbeitet. Die Trennung der honigartigen, meist nicht kristallisierenden Isomeren ist sehr aufwendig, sie kann auf dem erfindungsgemäß gewählten Syntheseweg später sehr einfach durchgeführt werden.

Anschließend wird das Halbestergemisch mit wäßrigem Alkali gemäß Schritt (3) zu dem isomeren Gemisch der Disäuren C verseift. Durch Erwärmen mit Acetylchlorid in Schritt (4) wird dann unter Wasserabspaltung das Isomerengemisch der cyclischen Anhydride D hergestellt.

Im Schritt (5) wird mit Aluminiumchlorid durch intramolekulare 5-Ring-Cyclisierung das Isomerengemisch E synthetisiert. Diese Verbindungen kristallisieren sehr gut und können in den meisten Fällen dank ihrer unterschiedlichen Löslichkeit in Benzol leicht in die beiden Isomeren getrennt werden.

Aus dem gewünschten Isomer entsteht unter dem Einfluß von Acetanhydrid durch intramolekulare 6-Ring-Cyclisierung gemäß Schritt (6) der Ester F. Dieser wird anschließend in Schritt (7) alkalisch zum Hydroxy-fluorenon-Derivat G verseift.

Die Umsetzung mit einem 2-Propin-1-ol-derivat (Schritt (8)) führt zu den schwach photochromen, stark dunkelrot gefärbten indenoannellierten Naphthopyranen H. Diese Umsetzung wird hier nicht näher beschrieben, da sie sich in analoger Weise wie bei den 1- oder 2-Naphtholen durchführen läßt. Beispiele hierzu finden sich u.a. in der US 5.066.818, der US 5.238.981 und der EP 0 246 114.

Mit entsprechenden Grignard-Verbindungen entstehen in Schritt (9) die Verbindungen J. Deren freie Hydroxylgruppe kann noch gemäß Schritt (10) zu der Verbindungsklasse K derivatisiert werden.

Der vorgenannte Syntheseweg weist nun gegenüber dem in der WO 96/14596 bzw. der US 5.645.767 empfohlenen Weg verschiedene Vorteile auf:

Zum einen sind verschiedene Verbindungen auf dem in der WO 96/14596 bzw. der US 5.645.767 beschriebenen Weg nicht darstellbar. Der erfindungsgemäß beschriebene Weg ist wesentlich schonender, so entfällt beispielsweise das einstündige Erhitzen mit konzentrierter Phosphorsäure auf über 190°C. Dadurch sind als Substituenten $R_1$ und $R_2$ auch empfindlichere Gruppierungen einsetzbar. Die Schließung des 5-Ringes vor dem 6-Ring im erfindungsgemäßen Verfahren führt ebenfalls häufig zu Produkten, die auf dem im Stand der Technik genannten Weg - Schließung des 6-Rings vor dem 5-Ring - nicht oder nur schwer darstellbar sind. Zum anderen gibt es selbst bei gleichem Zielprodukt, z.B. bei symmetrischen Benzophenonen als Ausgangsmaterial, nach dem erfindungsgemäß beschriebenen Verfahren weniger Nebenprodukte, bei einfacherer Reinigung und höheren Ausbeuten des photochromen Farbstoffs.

Bevorzugt können die photochromen Naphthopyran-Farbstoffe mehrere photochrome Farbzentren im Molekül enthalten, wobei das weitere photochrome Farbzentrum im kürzeren oder längeren Wellenlängenbereich absorbiert als das bereits vorhandene photochrome Farbzentrum. Beispielsweise kann im erfindungsgemäßen Verfahren das Grignard-Reagenz, mit dem die Umsetzung des Farbstoffs H in Schritt (9) erfolgt, oder das 2-Propin-1-ol-derivat, mit dem die Umsetzung des Farbstoffs G in Schritt (8) erfolgt, jeweils eine photochrome Verbindung enthalten.

Das genaue synthetische Vorgehen ist bei der Darstellung der Beispiele exemplarisch beschrieben.

Gegenstand der Erfindung ist auch die Verwendung der erfindungsgemäßen Naphthopyran-Farbstoffe. Diese können in Kunststoffen jeglicher Art und Form für eine Vielzahl von Einsatzzwecken, für die photochromes Verhalten von Bedeutung ist, Verwendung finden, wie bspw. in Linsen, Schutzgläsern, Visieren von Schutzhelmen, Fenstern, Brillengläsern, Schutzblenden, Abdekkungen, Dächern oder dergleichen.

Ferner betrifft die Erfindung auch einen photochromen Gegenstand, umfassend u.a. ein oder mehrere erfindungsgemäße photochrome Naphthopyran-Farbstoffe sowie mindestens ein Polymermaterial.

Beispiele für das im photochromen Gegenstand eingesetzte Polymermaterial sind Poly($C_1$-$C_{12}$-alkylmethacrylate), Poly(oxyalkylendimethacrylate), Poly (alkoxylierte Phenolmethacrylate), Celluloseacetat, Cellulosetriacetat, Celluloseacetatpropionat, Celluloseacetatbutyrat, Poly(vinylacetat), Poly(vinylalkohol), Poly(vinylchlorid), Poly (vinylidenchlorid), thermoplastische Polycarbonate, Polyester, Polyurethane, Poly(ethylenterephthalat), Polystyrol, Poly($\alpha$-methylstyrol), Copoly(styrolmethylmethacrylat), Copoly(styrolacrylonitril), Polyvinylbutyral. Ferner können auch Polymere der folgenden Monomere verwendet werden: Polyol(allylcarbonat)monomere, polyfunktionelle Acrylatmonomere, polyfunktionelle Methacrylatmonomere, Diethylenglycoldimethacrylatmonomere, ethoxylierte Bisphenol-A-dimethacrylatmonomere, Diisopropenylbenzolmonomere, Ethylenglycolbismethacrylatmonomere, Poly(ethylenglycol)-bismethacrylatmonomere, ethoxylierte Phenolmethacrylatmonomere, alkoxylierte Polyalkoholacrylatmonomere und Diallylidenpentaerythritolmonomere.

Erfindungsgemäß kann das Polymermaterial ein festes oder transparentes Homo- oder Copolymer sein, ausgewählt aus der Gruppe, bestehend aus Poly(methylmethacrylat), Poly(ethylenglycolbismethacrylat), poly(ethoxyliertem Bisphenol-A-dimethacrylat), thermoplastischem Polycarbonat, Poly(vinylacetat), Polyvinylbutyral, Polyurethan oder ein Polymer, ausgewählt aus den Bestandteilen der Gruppe, bestehend aus Diethylenglycolbis-(allylcarbonat)-monomeren, Diethylenglycoldimethacrylatmonomeren, ethoxylierten Phenolmethacrylatmonomeren, Diisopropenylbenzolmonomeren und ethoxylierten Trimethylolpropantriacrylatmonomeren.

Nach einer erfindungsgemäßen Ausführungsform kann der photochrome Gegenstand neben einem oder mehreren der oben beschriebenen Polymermaterialien und mindestens einem erfindungsgemäßen photochromen Naphthopyran-Farbstoff mindestens einen anderen organischen photochromen Farbstoff enthalten. Dieser zusätzliche Farbstoff besitzt bevorzugt zumindest ein aktiviertes Absorptionsmaximum im Bereich von etwa 400 bis 700 nm.

Der photochrome Gegenstand kann den oder die photochromen Naphthopyran-Farbstoffe in einer Menge von etwa 0,05 bis 2,5 mg pro $cm^2$ Polymermaterialoberfläche, in welche der oder die photochromen Farbstoffe eingebettet oder auf die sie aufgebracht sind, aufweisen.

Die erfindungsgemäßen photochromen Naphthopyran-Farbstoffe können im allgemeinen durch verschiedene, im Stand der Technik beschriebene Verfahren auf ein Polymermaterial, wie ein organisches Kunststoffmaterial, aufgebracht oder darin eingebettet werden, um zum photochromen Gegenstand der vorliegenden Erfindung zu gelangen. Man unterscheidet sogenannte Massefärbungs- und Oberflächenfärbungs-Verfahren.

Ein Massefärbungs-Verfahren umfaßt beispielsweise das Auflösen oder Dispergieren der photochromen Verbindung oder Verbindungen in einem Kunststoffmaterial, zum Beispiel durch die Zugabe der photochromen Verbindung (en) zu einem monomeren Material bevor die Polymerisation erfolgt. Eine weitere Möglichkeit zur Herstellung eines photochromen Gegenstands ist die Durchdringung des oder der Kunststoffmaterialien mit der (den) photochromen Verbindung(en) durch Eintauchen des Kunststoffmaterials in eine heiße Lösung des oder der photochromen Farbstoffe oder beispielsweise auch ein Thermotransferverfahren. Nach einer weiteren Verfahrensvariante kann die photochrome Verbindung in Form einer separaten Schicht zwischen aneinandergrenzenden Schichten des Kunststoffmaterials, z. B. als Teil eines polymeren Films, vorgesehen werden. Ferner ist auch ein Aufbringen der photochromen Verbindung (en) als Teil einer auf der Oberfläche des Kunststoffmaterials befindlichen Beschichtung möglich.

Der Ausdruck "Durchdringung" soll dabei die Migration einzig der photochromen Verbindung(en) in das Kunststoffmaterial, sei es durch den lösungsmittelunterstützten Transfer der photochromen Verbindung(en) in ein poröses Polymer, Dampfphasentransfer oder andere derartige Oberflächendiffusions-Vorgänge, bedeuten. Vorteilhafterweise können die photochromen Gegenstände der Erfindung, wie Brillengläser, nicht nur mittels der üblichen Massefärbung, sondern in gleicher Weise auch mittels Oberflächenfärbung hergestellt werden, wobei bei der letzteren Variante eine überraschend geringere Migrationsneigung erzielt werden kann. Dies ist vor allem bei nachfolgenden Veredelungsschritten von Vorteil, da - z.B. bei einer Antireflexbeschichtung durch die geringere Rückdiffusion im Vakuum - Schichtablösungen und ähnliche Defekte drastisch verringert werden.

Insgesamt können beliebig kompatible (in chemischer Hinsicht und farblicher Art und Weise verträgliche) Färbungen, d.h. Farbstoffe, auf das Kunststoffmaterial aufgebracht oder in es eingebettet werden, um sowohl ästhetischen Gesichtspunkten als auch medizinischen oder modischen Aspekten zu genügen. Der oder die spezifisch ausgewählten Farbstoffe können demzufolge, abhängig von den beabsichtigten Wirkungen sowie Anforderungen, variieren.

Die Eigenschaften der erfindungsgemäßen Verbindungen sollen anhand einiger Verbindungen erläutert werden, wobei die Erfindung selbstverständlich nicht auf diese Ausführungsformen beschränkt wird. Die besonderen Eigenschaften der erfindungsgemäß beanspruchten Molekülstrukturen können in Form der nachfolgend erläuterten speziellen Variationen einzeln oder in Kombination vorhanden sein, d.h. die gewünschten Eigenschaften sind entsprechend einstell- und variierbar und können als Einzeleigenschaften oder gleichzeitig vorliegen.

a.) Erfindungsgemäße 2H-Naphthopyrane mit nichtbasischen Aminosubstituenten

Die hervorragenden Eigenschaften der erfindungsgemäßen indenoannellierten 2H-Naphthopyrane, substituiert mit nichtbasischen Aminosubstituenten, zeigen sich bei der Herstellung von photochromen Kunststoffgläsern. Säurespuren, die bei der Hydrolyse des Gießharzes auftreten, attackieren diese Aminoverbindungen nicht. Ebenso weisen die Verbindungen eine im Vergleich deutlich bessere Lebensdauer auf als die aus dem Stand der Technik bekannten basischen Aminoverbindungen. Ein weiterer Vorteil bei vorliegen nichtbasischer Aminoverbindungen ist, wie bereits beschrieben, daß die Glashaftung nicht verringert wird, so daß eine Beeinträchtigung der Oberflächenbeschaffenheit, beispielsweise durch sog. Abplatzer, nicht auftritt.

Mittels Massefärbung wurden Kunststoffgläser hergestellt, die jeweils eine Menge von 500 ppm einer erfindungsgemäßen Verbindung und einer Verbindung aus dem Stand der Technik enthielten. Die Verbindungen hatten die folgenden Strukturen:

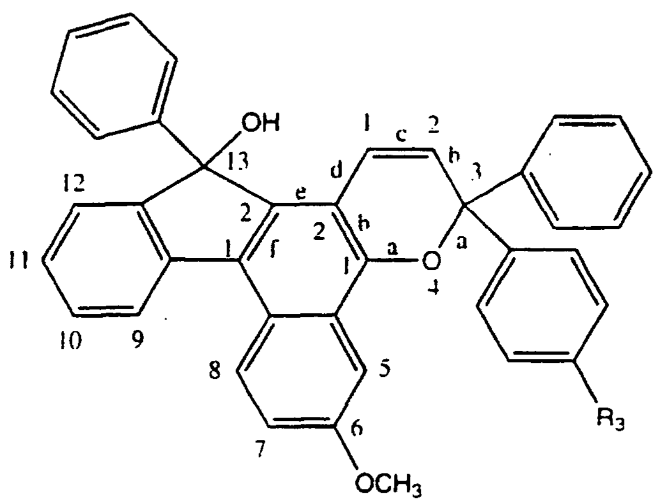

| Substituent | Beispiel | Stand der Technik |
|---|---|---|
| $R_3$ = $-OCH_3$ | 5 | Ja |
| $-NPh_2$ | 10 | Nein |

Von den beiden Verbindungen wurden Absorptionsspektren unter gleichen Bedingungen aufgenommen. Die Absorptionsspektren sind in Figur 3 abgebildet. Figur 3 zeigt einen Vergleich der Absorptionsbanden im Sichtbaren zwischen der Verbindung aus dem Stand der Technik und der erfindungsgemäßen Verbindung. Die Verbindungen in Figur 3 sind 13-Hydroxy-6-methoxy-3-(4-methoxyphenyl)-3,13-diphenyl-indeno[2,1-f]naphtho[1,2-b]pyran und 13-Hydroxy-6-methoxy-3-(4-diphenylaminophenyl)-3,13-diphenyl-indeno[2,1-f]naphtho[1,2-b]pyran.

Hierdurch wird belegt, daß die Verbindungen ein bathochrom verschobenes Absorptionsmaximum im Sichtbaren besitzen und hierdurch ausgezeichnete Absorptionseigenschaften zeigen. Die längstwellige Absorptionsbande, d.h. die Absorptionsbande zwischen dem roten bzw. infraroten Teil des Lichtspektrums und dem "Absorptionsloch" - der Stelle geringster Absorption im Bereich 400-500 nm - ist erfindungsgemäß sehr breit. Das Absorptionsverhalten der erfindungsgemäßen Verbindungen ist bei tiefer Eindunkelung (Transmission bei $\lambda_{max}$ < 10% ) bei 700 nm noch mindestens 50% und bei 750 nm mindestens 10% Absorption, wodurch die erfindungsgemäßen indenoannellierten 2H-Naphthopyrane den bekannten Verbindungen aus dem Stand der Technik weit überlegen sind.

b.) Erfindungsgemäße benzindeno-annellierte Naphthopyrane

Der Einsatz von Naphtho- anstelle von Benzophenonen als Ausgangsverbindungen A führt überraschenderweise zu längerwellig absorbierenden Verbindungen, sowohl in der geschlossenen Form (nicht angeregt; siehe Figur 4) als auch in der offenen (angeregten) Form (siehe Figur 5).

Die deutliche bathochrome Verschiebung und die drastische Erhöhung des Absorptionskoeffizienten der längstwelligen Absorptionsbande der geschlossenen Form ist auch für den Fachmann nicht zu erwarten, da der annellierte Naphthylring aufgrund der Verdrillung der beiden Ringsysteme durch sterische Hinderung der H-Atome, wie beim annellierien Phenylring, allenfalls einen geringen induktiven Effekt ausüben sollte. Die längstwellige Absorption wird weiterhin durch das Naphthopyran- und nicht durch das Naphthalinsystem verursacht. Da die spektrale Bestrahtungsstärke durch die Sonne auf der Erdoberfläche im Bereich 360-410 nm zu längeren Wellenlängen hin auf das 2,88 fache steigt (EN 1836, Tabelle C.1), ist dieser Effekt gerade für den Einsatz in phototropen Brillengläsern von besonderer Bedeutung.

Ebenso überraschend ist die bathochrome Verschiebung der Absorption der offenen Form. Moleküle mit dieser Struktureinheit absorbieren bereits weit in den roten Spektralbereich des sichtbaren Lichts hinein, der Farbeindruck ist deshalb grün. Mit den Verbindungen nach dem Stand der Technik ist demgegenüber - wie bereits beschrieben - nur der kürzerwellige Teil des Spektrums zugänglich, wobei der Farbeindruck orange bis blau/grau ist.

Um die oben erläuterte bathochrome Verschiebung der erfindungsgemäßen Verbindungen gegenüber dem Stand der Technik zu illustrieren, wurden Vergleichsversuche durchgeführt. Die Ergebnisse sind in der nachfolgenden Tabelle 1 zusammengestellt.

Die ersten drei Verbindungen aus Tabelle 1, V1, V2 und Beispiel 3, entsprechen dabei dem Stand der Technik (siehe WO 96 / 14596); die Herstellung von V1 und V2 wird dort im einzelnen beschrieben.

Die Herstellung der Probekörper und die Meßmethodik wird detailliert in den Beispielen beschrieben.

In den Figuren 4 und 5 sind die Absorption der geschlossenen Form bzw. die Absorption der offenen Form im Vergleich zur Absorption der Verbindungen V1 und V2 aus dem Stand der Technik, ausgedrückt durch Auftragung der Extinktion gegen die Wellenlänge, dargestellt. Bei der offenen Form wurde zum besseren Erkennen der bathochromen Verschiebung auf etwa gleiche Absorptionsintensität normiert.

Tabelle 1: Absorptionsmaxima der offenen und geschlossenen Form ausgehend von der Verbindung mit der allgemeinen Strukturformel (vgl. Figuren 4 und 5):

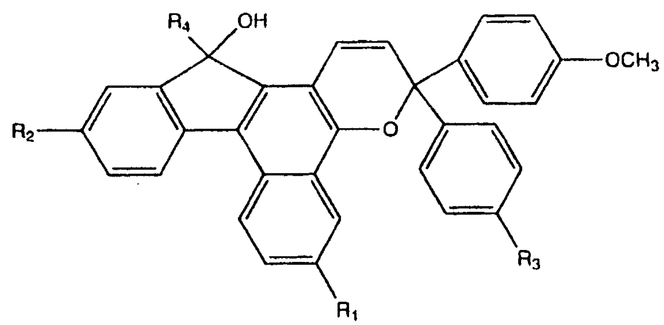

| Beispiel | Substituenten | | | | λmax (nm) | λmax (nm) | Farbe |
|---|---|---|---|---|---|---|---|
| | $R_1$ | $R_2$ | $R_3$ | $R_4$ | geschlossene Form | offene Form | |
| V1 | H | H | $OCH_3$ | H | 350 | 555 | violett |
| V2 | $CH_3$ | $CH_3$ | $OCH_3$ | H | 350,375 (Schulter) | 570 | blauviolett |
| 3 | $OCH_3$ | H | H | H | 360,375 (Schulter) | 575 | blau |
| 1 | $OCH_3$ | Benzo | H | H | 385 | 600 | grün |

c.) Erfindungsgemäße Verbindungen mit mehreren photochromen Farbzentren

Obwohl diese ringannellierten Naphthopyrane aufgrund ihrer Molekülstruktur bereits ein geringeres Migrationsverhalten als gewöhnliche Naphthopyrane aufweisen (vergleiche z.B. US 5.369.158), kann dieses durch Verdoppelung des Molekulargewichts noch weiter reduziert werden.

Dies ist auf zwei Wegen möglich. Über die Komponente X bzw. X', wie bereits in der WO 96/01884 dargestellt, oder über einen geeigneten Reaktionspartner R.

Wird beispielsweise 1,2-Bis-(4-bromphenyl)-ethan oder 1,2-Bis-(4-bromphenoxy)-ethan beidseitig zum Grignard umgesetzt und setzt man dann mit zwei Äquivalenten Naphthopyranen der Struktur H um, so entsteht ein symmetrisches Doppelmolekül. Ebenso ist es jedoch möglich, eine entsprechend substituierte phototrope Verbindung anderer Struktur, beispielsweise ein Spirooxazin oder ein kürzerwellig absorbierendes Naphthopyran, zum Grignard umzusetzen und mit den Naphthopyranen der Struktur H zu neuartigen Verbindungen umzusetzen. Wählt man diese zweite photochrome Verbindung so aus, daß deren Absorptionsmaximum gerade in das "Absorptionsloch" um 470 nm fällt, so erhält man in einer Molekülstruktur sehr stumpfe, nahezu neutrale Farben (grau - braun). Die Vorteile derartiger Verbindungen sind bereits in der DE 43 21 487 beschrieben. Beispielsweise sind durch Verdoppelung über den Substituenten X bzw. X' und nachfolgende nochmalige Verdoppelung über einen bifunktionellen Grignard Tetra-Systeme mit Molekulargewichten über 2000 zugänglich.

d.) Aufhellgeschwindigkeit der erfindungsgemäßen Naphthopyrane

Erfindungsgemäß wurde festgestellt, daß mit dem Einsatz von sterisch anspruchsvollen aromatischen Resten als $R_4$ eine signifikante und unerwartete Erhöhung der Aufhellgeschwindigkeit verbunden ist. Zugleich wird damit die Bildung von Fulvenen, die bei $\alpha$-Wasserstoff tragenden Alkylgruppen unter Wasserabspaltung entstehen können, unmöglich gemacht, so daß diese tieffarbigen Zerstörungsprodukte nicht mehr entstehen können.

Wird ein Alkylrest $R_4$, wie in der WO 96/14596 beschrieben, erfindungsgemäß durch Phenyl ersetzt, bringt dies bereits den oben beschriebenen Stabilitätsvorteil (keine Fulvenbildung). Die schnellere Aufhellung wird jedoch erst beim Einsatz sperrigerer Arylgruppen deutlich. Die Einführung von ortho-Substituenten in die Phenylringe bei X bzw. X' führt, wie in der US 5.066.818 beschrieben, zu einer starken Verlangsamung der Aufhellgeschwindigkeit. Die Einführung von ortho-Substituenten in den Phenylrest $R_4$ führt dagegen überraschenderweise zum Gegenteil, zu einer Erhöhung der Aufhellgeschwindigkeit. Besonders bevorzugt sind mehrfach substituierte Phenylreste bzw. deren analoge Arylreste wie 1- oder 2-Naphthyl, 9-Phenanthryl, Pyrenyl und Heteroaromaten. wie Benzofuranyl, Benzothienyl, Chinolyl oder Isochinolyl.

Dieser Effekt ist umso überraschender, da in der Alkylreihe die Einführung immer sperrigerer Reste keinen erkennbaren Effekt hat, wie aus Tabelle 2 zu ersehen.

Tabelle 2:

| Gemessene Aufhellgeschwindigkeit im Polymer CR 307 der Fa. PPG aus der WO 96/14596, S. 33<br>Substituenten : $R_1 = R_2 = CH_3$; $R_3 = OCH_3$, $R_4$ variabel | |
|---|---|
| $R_4$ | Aufhellgeschwindigkeit $T_{1/2}$ in sec. |
| $-CH_3$ | 362 |
| $-CH_2-CH_3$ | 237 |
| $-CH(CH_3)_2$ | 264 |
| $-C(CH_3)_3$ | 313 |
| $T_{1/2}$ = Zeit bis zur Aufhellung der Hälfte der Eindunkelung (Halbwertszeit) | |

Zur weiteren Veranschaulichung der obigen Darlegungen wurden wieder Vergleichsversuche mit den erfindungsgemäßen Verbindungen und bekannten Verbindungen durchgeführt. Die eingesetzten Verbindungen leiten sich dabei von der Verbindung mit der folgenden allgemeinen Formel ab:

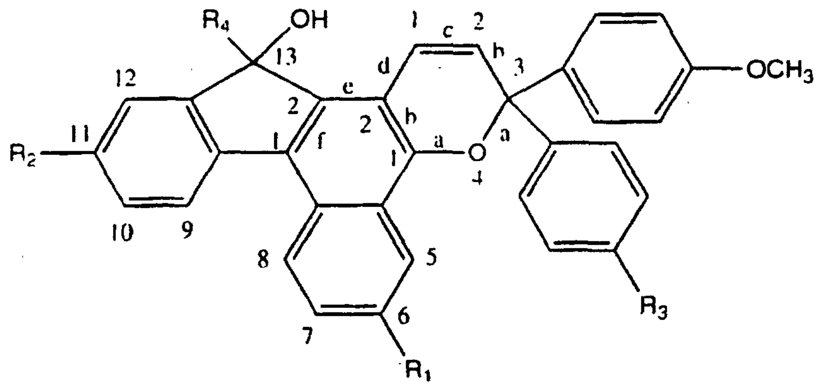

Es wurden die Aufhellgeschwindigkeit und die Aufhellkinetik im Polymer Transhade-150 der Fa. Tokuyama bei verschiedenen Temperaturen für verschieden substituierte Systeme gemessen. Die erhaltenen Ergebnisse sind nachfolgend zusammengestellt, wobei die folgenden Begriffe verwendet wurden:

$$\Delta = \text{relative Aufhellung binnen 10 min bei } 23°C$$

$$= 100\ \%\ (\tau 10min - \tau s) / (\tau o - \tau s)$$

$\tau s$ ist die nach 15 min Eindunkelung erreichte Transmission
$\tau o$ ist die Transmission im voll aufgehelltem Zustand
$\tau 10min$ ist die Transmission nach 10 min Aufhellung im Dunkeln

$T_{1/2}$ = Zeit bis zur Aufhellung der Hälfte der Eindunkelung (Halbwertszeit)

Die Aufhellkinetik verschieden substituierter Systeme wurde bestimmt, wobei $R_1$ , $R_2$ und $R_3$ entweder die Methoxygruppe oder Wasserstoff repräsentierten; die Messungen wurden bei 23°C und bei 40°C durchgeführt. Es wurden drei Versuchsreihen mit variablem $R_4$ gemessen; die Ergebnisse sind in den folgenden Tabellen 3 bis 5 zusammengefaßt.

Tabelle 3:

| Substituenten: $R_1 = OCH_3$; $R_2 = R_3 = H$ | | | |
|---|---|---|---|
| Substituent | Beispiel | Δ (10 min, 23° C) | $T_{1/2}$ (40°C) |
| $R_4 = -C_2H_5$ | 4 | 25 % | 7.0 min |
| $-C_6H_5$ | 5 | 22 % | 7.0 min |
| -1-naphthyl | 6 | 29 % | 5.0 min |
| -o-tolyl | 7 | 34 % | 4.2 min |
| -2-p-xylyl | 8 | 36 % | 4.0 min |
| -3-methyl-2-thienyl | 9 | 38 % | 3.4 min |

Tabelle 4:

| Substituenten: $R_1 = R_3 = OCH_3$; $R_2 = H$ | | | |
|---|---|---|---|
| Substituent | Beispiel | Δ (10 min, 23° C) | $T_{1/2}$ (40°C) |
| $R_4 = -C_4H_9$ | 11 | 34 % | 5.5 min |
| $-C_6H_5$ | 12 | 33 % | 5.6 min |

Tabelle 4: (fortgesetzt)

| Substituenten: $R_1 = R_3 = OCH_3$; $R_2 = H$ | | | |
|---|---|---|---|
| Substituent | Beispiel | Δ (10 min, 23° C) | $T_{1/2}$ (40°C) |
| -1-naphthyl | 13 | 40 % | 5.0 min |
| -o-tolyl | 14 | 43 % | 4.0 min |
| 2-p-xylyl | 15 | 44 % | 4.0 min |

Tabelle 5:

| Substituenten: $R_1 = R_2 = H$; $R_3 = OCH_3$ | | | |
|---|---|---|---|
| Substituent | Beispiel | Δ (10 min, 23° C) | $T_{1/2}$ (40°C) |
| $R_4$ = -H | V1 | 40 % | 2.7 min |
| $-CH_3$ | V3 | 43 % | 2.5 min |
| $-C_4H_9$ | V4 | 44 % | 2.5 min |
| $-C_6H_5$ | 16 | 44 % | 2.2 min |
| -2-p-xylyl | 17 | 51 % | 2.0 min |

Die Beispiele V1, V3, V4, 4, 5, 7, 11 sowie 12, 14 und 16 entsprechen dem Stand der Technik, wobei die Darstellung von V1, V3 und V4 in der WO 96/14596 explizit beschrieben ist.

Insgesamt sieht man aus den oben aufgeführten Tabellen 3 bis 5, daß in allen drei Versuchsreihen die Einführung von ortho-Substituenten im Arylrest $R_4$ eine signifikante Erhöhung der Aufhellgeschwindigkeit mit sich bringt.

Nachfolgend wird die Herstellung der einzelnen erfindungsgemäßen Naphthopyran-Farbstoffe in Beispielen detailliert erläutert, wobei diese Beispiele selbstverständlich den Schutzbereich der vorliegenden Erfindung nicht beschränken, sondern nur zur Veranschaulichung dienen sollen.

BEISPIEL 1

i) Aluminiumchlorid (40 g) wird in einem 1l-Kolben, der im Eisbad gut gekühlt wird, in etwa 400 ml Dichlormethan unter Rühren suspendiert. Anschließend gießt man langsam in dünnem Strahl geschmolzenes 2-Naphthoylchlorid (50 g; Schmelzpunkt 50°C) zu und läßt die Mischung (weiter im Eisbad) 5 min rühren. Dann tropft man eine Mischung aus Anisol (27 g) und 50 ml Dichlormethan zu und läßt die Reaktionsmischung unter Rühren über Nacht auftauen. Am nächsten Tag wird die Mischung unter Rühren auf 200 g Eis / 500 ml Wasser gegossen. Man rührt 10 min und trennt dann die organische Phase im Scheidetrichter ab. Diese wird zweimal mit Wasser extrahiert und über Natriumsulfat getrocknet. Nach Abdestillieren des Solvens kristallisiert das zurückbleibende Öl beim Stehenlassen. Nach Umkristallieren aus etwa 500 ml siedendem Methanol erhält man farblose Kristalle (58 g), die mittels NMR-Spektrum als 2-(4-Methoxybenzoyl)naphthalin identifiziert wurden.

ii) tert. Butanol wird im warmen Wasserbad vorab geschmolzen. Kalium-tert.butylat (30 g) wird in einem 1l-Dreihalskolben in 600 ml tert. Butanol suspendiert und obiges Reaktionsprodukt sowie Bernsteinsäuredimethylester (45 g) zugefügt. Die Mischung wird gut gerührt und 1 h unter Rückfluß erhitzt. Danach gibt man nochmals Kalium-tert.-butylat (30 g) sowie Bernsteinsäuredimethylester (45 g) zu und läßt 2 h unter Rückfluß erhitzen. Nach dem Abkühlen wird mit insgesamt 2 l Wasser hydrolysiert. Unter Rühren wird mit konz. Salzsäure angesäuert. Anschließend wird zweimal mit je 400 ml Diethylether extrahiert und die vereinigten Ether-Phasen werden einmal mit 400 ml Wasser gewaschen. Dann wird zweimal mit 500 ml gesättigter Natriumhydrogencarbonat-Lösung extrahiert. Dabei geht das Produkt als Carboxylat in die wäßrige Phase, die einmal mit 200 ml Ether gewaschen und anschließend in einem großen Becherglas unter Rühren mit konz. Salzsäure angesäuert wird. Anschließend wird zweimal mit je 400 ml Ether extrahiert und die organische Phase einmal mit Wasser gewaschen. Nach Trocknen über Natriumsulfat wird der Ether gründlich abrotiert. Das orangegelbe, honigartige Reaktionsprodukt (80 g) wurde mittels NMR-Spektrum als 3-Methoxycarbonyl-4-(4-methoxyphenyl )-4-(2-naphthyl)-3-butensäure identifiziert.

iii) In einem 1l-Kolben wird das obige Reaktionsprodukt in einer Lösung von Kaliumhydroxid (40 g) in etwa 600 ml Wasser aufgelöst. Die gebildete braune Reaktionslösung wird 3 h unter Rückfluß erhitzt. Nach dem Abkühlen wird unter Rühren und guter Kühlung im Eisbad mit konz. Salzsäure angesäuert. Anschließend wird zweimal mit je 400 ml Essigester extrahiert. Die vereinigten Essigester-Phasen werden einmal mit Wasser gewaschen und nach dem Trocknen über Natriumsulfat gründlich abrotiert. Das honigartige Reaktionsprodukt (75 g) wurde mittels NMR-Spektrum als 3-Carboxyl-4-(4-methoxyphenyl)-4-(2-naphthyl)-3-butensäure identifiziert.

iv) Das obige Reaktionsprodukt wird mit Acetylchlorid (35 g) in 300 ml Essigsäure gelöst. Unter Rühren wird nun 2 h unter Rückfluß erhitzt. Nach Abdestillieren des Solvens wird der Rückstand noch warm in etwa 600 ml Essigester gelöst und zweimal mit Wasser extrahiert. Anschließend wird zweimal mit je 300 ml 5%iger Natriumcarbonat-Lösung extrahiert. Die organische Phase wird nochmals mit Wasser gewaschen, über Natriumsulfat getrocknet und gründlich abrotiert. Das Reaktionsprodukt (65 g) fällt als dunkles, viskoses Öl an und wurde mittels NMR-Spektrum als (4-Methoxyphenyl-2-naphthylmethylen)bernsteinsäureanhydrid identifiziert.

v) Das obige Reaktionsprodukt wird in etwa 600 ml Dichlormethan bei Raumtemperatur gelöst und anschließend im Eisbad gut gekühlt. Anschließend wird unter gutem Rühren und Kühlen portionsweise Aluminiumchlorid (35 g) zugegeben und man läßt die Mischung über Nacht auftauen. Danach wird die Reaktionsmischung zur Hydrolyse in 1 l Eis/Wasser gegossen. Nach Abtrennen der organischen Phase wird diese zweimal mit je 500 ml Wasser gewaschen und anschließend zweimal mit 600 ml 5%iger Natronlauge extrahiert. Nach Waschen der vereinigten wäßrig-alkalischen Phasen mit 250 ml Ether wird mit konz. Salzsäure unter Rühren angesäuert. Es wird zweimal mit je 400 ml Essigester extrahiert und die organische Phase mit Wasser gewaschen, über Natriumsulfat getrocknet und gründlich abrotiert. Es hinterbleibt ein brauner fester Rückstand (45 g), der mittels NMR-Spektrum als 3-(4-Methoxyphenyl)-6,7-benzindenon-2-essigsäure identifiziert wurde. Das bei der Reaktion theoretisch ebenfalls entstehende Isomer 6-Methoxy-3-(2-naphthyl)-indenon-2-essigsäure konnte nicht detektiert werden.

vi) Das obige Reaktionsprodukt wird in etwa 300 ml Acetanhydrid suspendiert. Nach Zugabe von Natriumacetat (20 g) wird die Mischung 3 h unter Rühren zum Rückfluß erhitzt. Das Produkt fällt beim Abkühlen dick aus. Nach Abkühlen auf Raumtemperatur (und noch kurz im Kühlschrank) wird der gebildete weiche, kristalline, orangebräunliche Niederschlag abgesaugt und mit wenig Acetanhydrid gewaschen, bis das Filtrat nicht mehr dunkelbraun abläuft. Anschließend wird gründlich mit Wasser gewaschen und bei 60° C getrocknet. Das Produkt (leuchtend orangefarbener Feststoff; 30 g) wurde mittels NMR-Spektrum als 11-Acetoxy-9-methoxy-dibenzo[a,g]fluoren-13-on identifiziert.

vii) Das obige Reaktionsprodukt wird in 400 ml Ethanol suspendiert und mit Kaliumhydroxid (25 g) versetzt. Die Reaktionsmischung wird 1,5 h unter Rühren zum Rückfluß erhitzt, wobei sich allmählich eine prächtige tiefgrüne Farbe beobachten läßt. Nach Abkühlen wird etwa die Hälfte des Ethanols abrotiert und der verbleibende Rückstand zusammen mit 1 l Wasser auf der Heizplatte erhitzt. bis sich eine tiefgrüne Lösung gebildet hat. Dann wird die Lösung von der Heizplatte genommen und noch heiß unter Rühren mit konz. Salzsäure angesäuert. Es entsteht eine umbrabraune Suspension, die unter Rühren auf Raumtemperatur abgekühlt wird. Die Suspension wird abgesaugt und sorgfältig mit Wasser gewaschen. Der abgenutschte Stoff ist noch sehr schlammig und wird so gut wie möglich an der Membranpumpe trockengesaugt. Nach dem Trocknen bei 60°C wurde das umbrafarbene Produkt (25 g) mittels NMR-Spektrum als 11-Hydroxy-9-methoxy-dibenzo[a,g]fluoren-13-on identifziert.

viii) 3 g des obigen Reaktionsproduktes werden zusammen mit 4-Methoxybenzophenonacetylid (4 g; hergestellt aus 4-Methoxybenzophenon und Natriumacetylid) in etwa 300 ml Toluol suspendiert. Nach Zusatz einer Spatelspitze 4-Toluolsulfonsäure wird die Reaktionsmischung 1,5 h unter Rückfluß erhitzt. Im Laufe der Reaktion geht das zunächst schlechtlösliche Naphthol-Edukt immer mehr in Lösung; es entsteht eine rotbraune Mischung. Nach kurzem Abkühlen wird das Toluol im Vakuum abrotiert und der Rückstand in 40 ml Dichlormethan gelöst und einer Säulenchromatographie mit Aluminiumoxid (Wassergehalt 3%) als stationärer und Dichlormethan/Hexan-Mischung (2:1) als mobiler Phase unterzogen. Zur endgültigen Reinigung wird das Rohprodukt in etwa 100 ml Methanol digeriert und leicht erwärmt. Nach Abkühlen wird die entstandene Suspension abgesaugt, mit Methanol gewaschen und getrocknet. Das dunkle Produkt (3 g) wurde mittels NMR-Spektrum als 6-Methoxy-3-(4-methoxyphenyl)-3-phenyl-13-oxo-benz[p]indeno[2,1-f]-naphtho[1,2-b]pyran identifiziert.

ix) 1 g des obigen Reaktionsproduktes wird in 50 ml absolutem THF unter Rühren gelöst und im Eisbad abgekühlt. Man gibt solange Lithiumaluminiumhydrid in kleinen Portionen zu, bis sich die Farbe der Reaktionslösung nicht mehr weiter aufhellt. Man rührt noch 10 min. und gießt in Wasser, säuert mit konz. Salzsäure solange an, bis die Phasen klar sind und trennt die organische Phase ab. Nach Extraktion mit Wasser, Trocknen über Natriumsulfat

und Abrotieren des Solvens hinterbleibt ein bräunlicher viskoser Rückstand, der durch Zugabe von Methanol kristallisiert. Man rührt die Suspension noch einige Minuten bei Raumtemperatur und saugt dann den Niederschlag ab. Der leicht bräunliche Feststoff (0.8 g) wurde mittels NMR-Spektrum als 6-Methoxy-3-(4-methoxyphenyl)-3-phenyl-13-hydroxy-benz[p]indeno[2,1-f]naphtho[1,2-b]pyran identifiziert. In Lösung sowie im Glas ist grüne Photochromie zu beobachten.

BEISPIEL 2

Die Durchführung erfolgt analog Beispiel 1, nur im Schritt ix) werden 1 g des Reaktionsproduktes aus Schritt viii) in 50 ml absolutem THF bei Raumtemperatur unter Rühren gelöst. Zu dieser Lösung werden 2 Äquivalente Phenylmagnesiumchlorid-Lösung zugegeben und das ganze bei Raumtemperatur 1h gerührt. Anschließend gießt man in Wasser, säuert mit konz. Salzsäure solange an, bis die Phasen klar sind und trennt die organische Phase ab. Nach Extraktion mit Wasser, Trocknen über Natriumsulfat und Abrotieren des Solvens hinterbleibt ein dunkelbraunes Öl, das durch Zugabe von Methanol kristallisiert. Man rührt die Suspension noch einige Minuten bei Raumtemperatur und saugt dann den Niederschlag ab. Der beigefarbene Feststoff (0.7 g) wurde mittels NMR-Spektrum als 6-Methoxy-3-(4-methoxyphenyl)-3,13-diphenyl-13-hydroxy-benz[p]indeno[2,1-f]naphtho[1,2-b]pyran identifiziert.

BEISPIEL 3

Die Schritte i) bis vi) dieses Beispiels wurden auf der Grundlage des im J.Chem.Soc.1958, S. 986 von F.G. Baddar, L.S.El-Assal und V.B.Baghos publizierten Artikels mit einigen Modifikationen durchgeführt.

i) bis vi) Analog Beispiel 1 Schritt ii) mit 4-Methoxybenzophenon (50 g) als Ausgangsmaterial. Es entsteht 3-Methoxycarbonyl-4-(4-methoxyphenyl)-4-phenyl-3-butensäure (75 g), die analog Beispiel 1 Schritte iii) bis v) weiter zu einem Gemisch aus 3-(4-Methoxyphenyl)-indenon-2-essigsäure und 6-Methoxy-3-phenyl-indenon-2-essigsäure verarbeitet wird. Die Isomerentrennung gelingt durch heißes Digerieren in Benzol. Nach Abkühlen und Absaugen der Suspension erhält man als Rückstand reine 3-(4-Methoxyphenyl)-indenon-2-essigsäure als orangegelbe Kristalle (25 g), die mittels NMR-Spektrum identifiziert wurde. Diese wurde analog Beispiel 1 Schritte vi) bis vii) umgesetzt zu 5-Hydroxy-3-methoxy-7H-benzo[c]fluoren-7-on (20 g), das als rotviotettes Pulver anfiel und mittels NMR-Spektrum identifiziert wurde.

vii) Analog Beispiel 1 Schritt viii) mit 3 h Erhitzen bei 100° C statt Refluxen ergibt 6-Methoxy-3-(4-methoxyphenyl)-3-phenyl-13-oxo-indeno[2,1-f]naphtho[1,2-b]pyran als dunkelroten Feststoff (3g), der mittels NMR-Spektrum identifiziert wurde.

viii) Die analoge Umsetzung zu Beispiel 1 Schritt ix) mit obigem Produkt liefert 6-Methoxy-3-(4-methoxyphenyl)-3-phenyl-13-hydroxy-indeno[2,1-f]naphtho-[1,2-b]pyran (beiges Pulver; 0,7 g), das mittels NMR-Spektrum identifiziert wurde.

BEISPIEL 4

Die Durchführung erfolgt analog Beispiel 3, nur im Schritt viii) wird 1 g des Reaktionsproduktes aus Schritt vii) in 50 ml absolutem THF unter Rühren gelöst. Zu dieser Lösung werden 2 Äquivalente Ethylmagnesiumchlorid-Lösung zugegeben und das ganze bei Raumtemperatur 1 h gerührt. Anschließend gießt man in Wasser, säuert mit konz. Salzsäure solange an, bis die Phasen klar sind und trennt die organische Phase ab. Nach Extraktion mit Wasser, Trocknen über Natriumsulfat und Abrotieren des Solvens hinterbleibt ein dunkelbraunes Öl, das durch Zugabe von Methanol kristallisiert. Kristallisiert das Produkt nicht, wird solange im Eisbad gerührt, bis Kristalle ausfallen. Man rührt die Suspension noch einige Minuten bei Raumtemperatur, saugt dann den Niederschlag ab und wäscht mit Methanol nach. Der entstandene bräunliche Feststoff (0.5 g) wurde mittels NMR-Spektrum als 6-Methoxy-3-(4-methoxyphenyl)-3-phenyl-13-ethyl-13-hydroxy-indeno[2,1-f]-naphtho[1,2-b]pyran identifiziert.

BEISPIELE 5-9

Die Durchführung erfolgt analog Beispiel 4, nur statt Ethylmagnesiumchlorid
im Beispiel 5 Phenylmagnesiumchlorid,
im Beispiel 6 1-Naphthylmagnesiumbromid
im Beispiel 7 2-Methylphenylmagnesiumbromid
im Beispiel 8 2,5-Dimethylphenylmagnesiumbromid und im Beispiel 9 3-Methyl-2-thienylmagnesiumbromid.

Die Produkte (meist beigefarbene Pulver) wurden mittels NMR-Spektren als die am C-13 mit dem entsprechenden Grignardrest substituierten 6-Methoxy-3-(4-methoxyphenyl)-3-phenyl-13-hydroxy-indeno[2,1-f]naphtho [1,2-b]pyrane identifiziert.

BEISPIEL 10

Die Durchführung erfolgt analog Beispiel 3, nur im Schritt vii) erfolgt die Umsetzung mit 4-Diphenylaminobenzophenonacetylid [hergestellt aus 4-Diphenylaminobenzophenon (B. Staskun, J. Org. Chem., 1968, 33, 3031) und Natriumacetylid] statt 4-Methoxybenzophenonacetylid. Es wird 3 h unter Rückfluß erhitzt. Es entsteht 6-Methoxy-3-phenyl-3-(4-diphenylaminophenyl)-13-oxo-indeno [2,1-f]naphto[1,2-b]pyran als dunkelroter Feststoff, der mittels NMR-Spektrum identifiziert wurde. Dieses wird analog Beispiel 4 im Schritt viii) mit Phenylmagnesiumchlorid-Lösung (analog Beispiel 2) statt Ethylmagnesiumchlorid-Lösung umgesetzt. Man erhält 6-Methoxy-3,13-diphenyl-3-(4-diphenylaminophenyl)-indeno[2,1-f]naphtho[1,2-b]pyran als hellgrauen Feststoff, der mittels NMR-Spektrum identifiziert wurde.

BEISPIEL 11

Die Durchführung erfolgt analog Beispiel 4, nur mit 6-Methoxy-3,3-bis(4-methoxyphenyl)-13-oxo-indeno[2,1-f] naphtho[1,2-b]pyran als Ausgangsmaterial statt 6-Methoxy-3-(4-methoxy-phenyl)-3-phenyl-13-oxo-indeno[2,1-f]naphtho[1,2-b]pyran. Es entsteht 6-Methoxy-3,3-bis(4-methoxyphenyl)-13-butyl-13-hydroxy-indeno[2,1-f]naphto[1,2-b]pyran (0.6 g) als bräunlicher Feststoff, der mittels NMR-Spektrum identifiziert wurde.

BEISPIELE 12-15

Die Durchführung erfolgt analog Beispiel 11, nur statt Butylmagnesiumchlorid im Beispiel 12 Phenylmagnesiumchlorid,
im Beispiel 13 1-Naphthylmagnesiumbromid
im Beispiel 14 2-Methylphenylmagnesiumbromid und im Beispiel 15 2,5-Dimethylphenylmagnesiumbromid.

Die Produkte (meist beigefarbene Pulver) wurden mittels NMR-Spektren als die am C-13 mit dem entsprechenden Grignardrest substituierten 6-Methoxy-3,3-bis(4-methoxyphenyl)13-hydroxy-indeno[2,1-f]naphtho[1,2-b]pyrane identifiziert.

BEISPIEL 16

Die Durchführung erfolgt analog Beispiel 1 Schritte ii) bis ix), jedoch im Schritt ii) mit Benzophenon als Ausgangsmaterial, im Schritt viii) mit 4,4'-Dimethoxybenzophenonacetylid statt 4-Methoxybenzophenonacetylid und im Schritt ix) mit Phenylmagnesiumchlorid-Lösung (analog Beispiel 2) statt Lithiumaluminiumhydrid. Man erhält als bräunlichen Feststoff 3,3-Bis(4-methoxyphenyl)-13-hydroxy-13-phenyl-indeno[2,1-f]naphto[1,2-b]pyran (0.6 g), der mittels NMR-Spektrum identifiziert wurde.

BEISPIEL 17

Die Durchführung erfolgt analog Beispiel 16 mit 2,5-Dimethylphenylmagnesiumbromid statt Phenylmagnesiumchlorid. Man isoliert 3,3-Bis(4-methoxyphenyl)-13-(2,5-dimethylphenyl)-13-hydroxy-indeno[2,1-f]naphtho[1,2-b]pyran (0.5 g) als beigen Feststoff, der mittels NMR-Spektrum identifiziert wurde.

Herstellung der Probekörper:

500 ppm des jeweiligen photochromen Farbstoffes werden im verwendeten Monomer (TRANSHADE-150 der Firma Tokuyama; Brechungsindex 1,52) bei Raumtemperatur unter Rühren gelöst. Nach Zugabe eines Initiators vom Alkylperoxyester-Typ (1.5 Gew.-%) wird zweimal entgast und anschließend nach dem von der Firma Tokuyama empfohlenen Temperaturprogramm polymerisiert. Die Glasgießformen sind schwarz gefärbt, damit die Gesamtheit der photochromen Farbstoffe im nichtangeregten Zustand in die Matrix eingebaut werden kann. Nach Beendigung der Polymerisation werden die Probekörper noch 2 h bei 100°C getempert.

Aufnahme der Kinetikkurven:

Zur Ermittlung der Aufhellgeschwindigkeiten wurden die hergestellten Probekörper in einer üblichen Kinetikbank vermessen (Bestrahlung mit 50 klux gemäß EN 1836 Punkt 6.1.3.1.1.). Die Belichtungszeit beträgt jeweils 15 min, die Aufhellung findet im Dunkeln statt. Während Belichtung und Aufhellung wird die Transmission - bewertet nach der Hellempfindlichkeit des menschlichen Auges V λ - aufgezeichnet. Die Temperatur des Glases wird über eine thermostatisierbare Küvette geregelt.

## Patentansprüche

**1.** Photochrome Naphthopyran-Farbstoffe mit der allgemeinen Strukturformel (I)

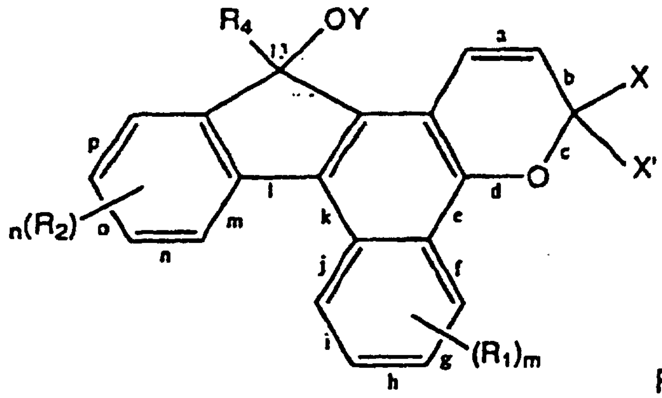

Formel (I)

wobei

$R_1$ und $R_2$ unabhängig voneinander gleich oder verschieden sind und ausgewählt sind aus

der Gruppe f, bestehend aus ($C_1$-$C_6$)-Alkyl, ($C_1$-$C_6$)-Alkoxy, Chloro und Fluoro;

oder

der Gruppe, bestehend aus Wasserstoff, ($C_5$-$C_6$)-Cycloalkyl, Phenyl, Benzyl, Dialkyl-($C_1$-$C_6$)-amino, Dicyclohexylamino, Diphenylamino, Piperidyl, Morpholinyl und Pyridyl, wobei m, n = 0, 1 oder 2 sind;

oder

zwei $R_1$ und/oder zwei $R_2$ bilden zusammen, unabhängig voneinander, einen carbo- oder heterocyclischen Ring, ausgewählt aus Benzol, Pyridin, Pyrazin, Pyrimidin, Furan und Thiophen;

$R_4$ ausgewählt ist aus

der Gruppe g, bestehend aus ($C_5$-$C_6$)-Cycloalkyl, ($C_1$-$C_6$)-Acyl, ($C_1$-$C_6$)-Alkoxy, Phenyl, Benzyl, monosubstituiertem Phenyl, monosubstituiertem Benzyl, wobei die Arylsubstituenten ($C_1$-$C_6$)-Alkyl oder ($C_1$-$C_6$)-Alkoxy sind;

oder aus

monochlor- oder monofloursubstituiertes Phenyl, Naphtyl, Phenanthryl, Pyrenyl, Chinolyl, Isochinolyl, Benzofuranyl, Benzothienyl, Dibenzofuranyl, Dibenzothienyl, Carbazolyl, oder Indolyl, jeweils unsubstituiert oder monosubstituiert, wobei die Substituenten ausgewählt sind aus der Gruppe bestehend aus Chloro, Fluoro, ($C_1$-$C_6$)-Alkyl oder ($C_1$-$C_6$)-Alkoxy;

oder aus

di- oder trisubstituierten Phenyl ausgewählt sind aus der Gruppe bestehend aus Chloro, Fluoro, ($C_1$-$C_6$)- Alkyl, ($C_1$-$C_6$)- Alkoxy, ($C_1$-$C_6$)-ω-Phenylalkyl und ($C_1$-$C_6$)-ω-Phenoxyalkyl, wobei der ω-ständige Phenylring kann dabei wiederum den X, X' oder $R_4$-Rest eines weiteren photochromen Pyransystems darstellen kann;

X und X' unabhängig voneinander ausgewählt sind aus der Gruppe z, bestehend aus a) und b) mit

a) den un-, mono-, di- und trisubstituierten Arylgruppen Phenyl und Naphthyl und
b) den un-, mono-, di- und trisubstituierten heterocyclischen Gruppen Pyridyl, Furanyl, Benzofuran-2-yl, Benzofuran-3-yl, Thienyl, Benzothien-2-yl und Benzothien-3-yl,

und die Substituenten in a) und b) ausgewählt sind aus

der Gruppe h, bestehend aus Hydroxy, Amino, Mono-($C_1$-$C_6$)-alkylamino, Di-($C_1$-$C_6$)-alkylamino, Piperidino, Morpholino, Pyrryl, ($C_1$-$C_6$)-Alkyl, ($C_1$-$C_6$)-Chloralkyl, ($C_1$-$C_6$)-Fluoralkyl, ($C_1$-$C_6$)-Alkoxy, Mono-($C_1$-$C_6$)-alkoxy-($C_1$-$C_4$)-alkyl, Chloro und Fluoro; oder

der Gruppe a, bestehend aus nichtbasischen Aminen, wie unsubstituiertem oder substituiertem Diarylamino, Pyrazol, Imidazol, Indol, Pyridin, Pyrazolin, Imidazolin, Pyrrolin, Phenothiazin, Phenoxazin, Phenazin, Acridin oder Carbazol, wobei die Substituenten an den nichtbasischen Aminen ausgewählt sind aus ($C_1$-$C_4$)-Alkyl, ($C_1$-$C_4$)-Alkoxy, Phenyl, Fluoro, Chloro und Bromo;

oder ausgewählt sind aus .

c) ($C_1$-$C_6$)-Alkyl, ($C_1$-$C_6$)-Chloralkyl, ($C_1$-$C_6$)-Fluoralkyl, Mono-($C_1$-$C_6$)-alkoxy-($C_1$-$C_4$)-alkyl, ($C_3$-$C_6$)-Cycloalkyl, Mono-($C_1$-$C_6$)-alkoxy-($C_3$-$C_6$)-cycloalkyl, Chlor-($C_3$-$C_6$)-cycloalkyl und Fluor-($C_3$-$C_6$)-cycloalkyl;

oder

X und X' bilden mit dem Kohlenstoffatom der Verknüpfungsstelle mit dem Pyran einen gesättigten $C_5$-$C_{10}$ monocyclischen, $C_7$-$C_{12}$ bicyclischen oder $C_7$-$C_{12}$ tricyclischen Kohlenwasserstoffring oder ein Fluoren-9-yliden;

oder

entweder X oder X' ist mono- oder disubstituiertes Phenyl, das in para-Stellung zur Verknüpfung mit -Phenyl, $-(CH_2)_p$-Phenyl oder $-O-(CH_2)_p-O$-Phenyl substituiert ist (p = 1, 2, 3...,6), wobei der zweite Phenylring zu einem zweiten - nicht notwendigerweise identischen - photochromen Pyran gehört;

und

Y ausgewählt ist aus der Gruppe, bestehend aus Wasserstoff, ($C_1$-$C_6$)-Alkyl, ($C_1$-$C_6$)-Acyl, unsubstituiertem Benzoyl, unsubstituiertem Naphthoyl, monosubstituiertem Benzoyl und monosubstituiertem Naphthoyl, wobei die Arylsubstituenten ($C_1$-$C_6$)-Alkyl oder ($C_1$-$C_6$)-Alkoxy sind;

mit der Maßgabe, daß $R_1$ und $R_2$ nicht aus der Gruppe f sind, wenn $R_4$ aus der Gruppe g und X und X' aus der Gruppe z sind, für den Fall, daß die Reste der Gruppe z unsubstituiert oder die Substituenten aus der Gruppe h sind.

2. Photochrome Naphthopyran-Farbstoffe nach Anspruch 1, **dadurch gekennzeichnet, daß** die Heterocyclen in der Gruppe $R_4$ über den Carbocyclus mit dem Naphthopyransystem verbunden sind.

3. Photochrome Naphthopyran-Farbstoffe nach Anspruch 1 oder 2, **dadurch gekennzeichnet, daß** $R_1$, $R_2$, $R_4$ und Y wie in Anspruch 1 definiert sind und X und X' ausgewählt sind aus der in Anspruch 1 definierten Gruppe z,
mit der Maßgabe, daß $R_1$ und $R_2$ nicht aus der Gruppe f sind, wenn $R_4$ aus der Gruppe g und X und X' aus der Gruppe z sind, für den Fall, daß die Reste der Gruppe z unsubstituiert oder die Substituenten aus der Gruppe h sind.

4. Photochrome Naphthopyran-Farbstoffe nach einem der Ansprüche 1, 2 oder 3, **dadurch gekennzeichnet, daß** zwei $R_1$ und/oder zwei $R_2$ unabhängig voneinander einen carbo- oder heterocyclischen Ring, ausgewählt aus Benzol, Pyridin, Pyrazin, Pyrimidin, Furan und Thiophen bilden.

5. Photochrome Naphthopyran-Farbstoffe nach einem der Ansprüche 1 bis 4, **dadurch gekennzeichnet, daß** mindestens ein X oder X' einen zumindest mit einem nichtbasischen Amin aus der Gruppe a substituierten, oben definierten Rest aus der Gruppe z darstellt.

6. Photochrome Naphthopyran-Farbstoffe nach Anspruch 5, **dadurch gekennzeichnet, daß** X und X' die gleichen Reste aus der Gruppe z mit unterschiedlichen Substituenten darstellen.

7. Photochrome Naphthopyran-Farbstoffe nach Anspruch 5 oder 6, **dadurch gekennzeichnet, daß** die Substituenten an X und X' gleich sind.

8. Photochrome Naphthopyran-Farbstoffe nach einem der Ansprüche 1 bis 3. **dadurch gekennzeichnet, daß** $R_4$ ausgewählt ist aus der Gruppe, bestehend aus zumindest in ortho-Position substituierten Phenylresten, 1- oder 2-Naphthyl, 9-Phenanthryl, Pyrenyl und Heteroaromaten, wie Benzofuranyl, Benzothienyl, Chinolyl oder Isochinolyl.

**9.** Photochrome Naphthopyran-Farbstoffe nach einem der Ansprüche 1 bis 8. **dadurch gekennzeichnet, daß** eine oder mehrere Methoxygruppen in 5-, 6-, 7- und/oder 8-Position des Naphthopyranrings vorhanden sind.

**10.** Photochrome Naphthopyran-Farbstoffe nach einem der Ansprüche 1 bis 9, **dadurch gekennzeichnet, daß** der photochrome Farbstoff mehrere photochrome Farbzentren im Molekül enthält.

**11.** Photochrome Naphthopyran-Farbstoffe nach Anspruch 10, **dadurch gekennzeichnet, daß** das weitere photochrome Farbzentrum im kürzeren oder längeren Wellenlängenbereich absorbiert als das bereits vorhandene photochrome Farbzentrum.

**12.** Photochrome Naphthopyran-Farbstoffe nach einem der vorangehenden Ansprüche, **dadurch gekennzeichnet, daß** die Eigenschaften der Farbstoffe, wie Absorption, Migration und Aufhellgeschwindigkeit durch die Auswahl der Substituenten gezielt eingestellt werden können.

**13.** Photochrome Naphthopyran-Farbstoffe nach einem der vorangehenden Ansprüche, **dadurch gekennzeichnet, daß** das Molekulargewicht des Farbstoffs durch Auswahl der Substituenten eingestellt und/oder vervielfacht werden kann.

**14.** Photochrome Naphthopyran-Farbstoffe mit der nachfolgenden chemischen Strukturformel (II):

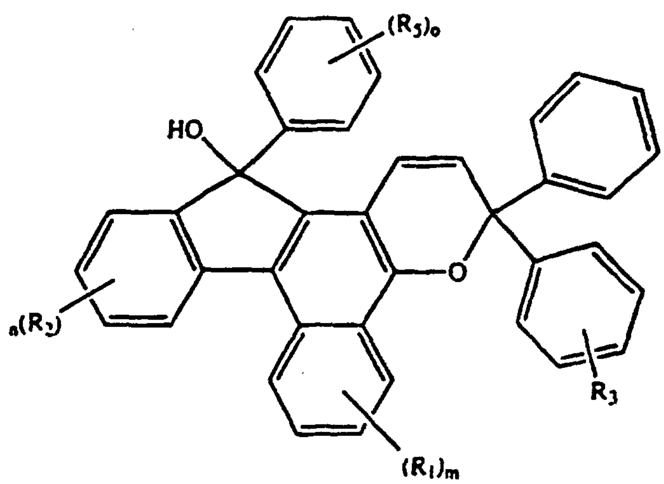

Formel (II)

wobei
$R_1$, $R_2$, m und n wie in Anspruch 1 definiert sind,
$R_5$ die für $R_4$ = Phenyl definierten Substituenten mit o = 1, 2 oder 3 darstellt und
$R_3$ ein nichtbasisches Amin aus der in Anspruch 1 definierten Gruppe a ist.

**15.** Photochrome Naphthopyran-Farbstoffe nach Anspruch 14, **dadurch gekennzeichnet, daß** der nichtbasische Aminosubstituent unsubstituiertes oder substituiertes Diarylamino ist, und zumindest ein Arylring mono-, dioder trisubstituiert sein kann, wobei der oder die Substituenten ausgewählt sind aus ($C_1$-$C_4$)-Alkyl, ($C_1$-$C_4$)-Alkoxy, Phenyl, Fluoro, Chloro und Bromo.

**16.** Photochromer Naphthopyran-Farbstoff ausgewählt aus der Gruppe bestehend aus

1) 6-Methoxy-3-(4-methoxyphenyl)-3,13-diphenyl-13-hydroxybenz[p]indeno[2,1-f]naphtho[1,2-b]pyran,
2) 6-Methoxy-3-(4-methoxyphenyl)-3-phenyl-13-(2,5-dimethylphenyl)-13-hydroxybenz[p]indeno[2,1-f]naphtho[1,2-b]pyran,
3) 3-(4-Diphenylaminophenyl)-3-phenyl-13-(2,5-dimethylphenyl)-13-hydroxy-benz[p]indeno[2,1-f]naphtho [1,2-b]pyran,
4) 3-(4-Diphenylaminophenyl)-6-methoxy-3,13-diphenyl-13-hydroxyindeno[2,1-f]naphtho[1,2-b]pyran,
5) 3-(4-Diphenylaminophenyl)-6-methoxy-3-phenyl-13-(2,5-dimethylphenyl)-13-hydroxy-indeno[2,1-f]naphtho[1,2-b]pyran,
6) 6-Methoxy-3-(4-methoxyphenyl)-3-phenyl-13-(2,5-dimethylphenyl)-13-hydroxyindeno[2,1-f]naphtho[1,2-b]pyran,
7) 6-Methoxy-3-(4-methoxyphenyl)-3-phenyl-13-(1-naphthyl)-13-hydroxy-indeno[2,1-f]naphtho[1,2-b]pyran,
8) 6,11-Dimethoxy-3-(4-methoxyphenyl)-3-phenyl-13-(2,5-dimethylphenyl)-13-hydroxy-indeno[2,1-f]naphtho[1,2-b]pyran,
9) 3-(4-Methoxyphenyl)-3-phenyl-13-(2,5-dimethylphenyl)-13-hydroxyindeno[2,1-f]naphtho[1,2-b]pyran und
10) 6-Methoxy-3-(4-methoxyphenyl)-3-phenyl-13-(3-methyl-2-thienyl)-13-hydroxyindeno[2,1-f]naphtho[1,2-b]pyran.

**17.** Verfahren zur Herstellung der photochromen Naphthopyran-Farbstoffe nach einem der Ansprüche 1 bis 16, **gekennzeichnet durch** die folgenden Verfahrensschritte (1) bis (10):

(1) Umsetzung substituierter oder unsubstituierter Benzol- oder Naphthalinverbindungen **durch** Friedel-Crafts-Acylierung zu Benzo- oder Naphthophenonen A;

(2) Durchführung einer Stobbe-Kondensation der Verbindung A mittels Bernsteinsäurediester zum Isomerengemisch der Halbester B;

(3) Verseifung der Halbester B mit wäßrigem Alkali zum isomeren Gemisch der Disäuren C;

(4) Erwärmen der Disäuren C mit Acetylchlorid unter Wasserabspaltung zum Isomerengemisch der cyclischen Anhydride D;

(5) Umsetzung der Anhydride D mit Aluminiumchlorid unter intramolekularer 5-Ring-Cyclisierung zum Isomerengemisch E und Trennung der Isomeren mittels Löslichkeitsfraktionierung in Benzol;

(6) Umsetzung des entsprechenden Isomeren mit Acetanhydrid unter intramolekularer 6-Ring-Cyclisierung zum Ester F.

(7) Alkalische Verseifung des Esters F zum Hydroxy-fluorenon-Derivat G;

(8) Umsetzung der Verbindung G mit einem 2-Propin-1-ol-derivat zum photochromen indenoannellierten Naphthopyran-Farbstoff H;

(9) Grignard-Umsetzung des Farbstoffs H mit der Verbindung $R_4MgHal$ zur Verbindung J, wobei $R_4$ die oben genannte Bedeutung besitzt und Hal ein Halogenidion ist; und

(10) Derivatisierung der freien Hydroxylgruppe der Verbindung J zur Verbindung K.

**18.** Verfahren nach Anspruch 17, **dadurch gekennzeichnet, daß** das Grignard-Reagenz, mit dem die Umsetzung des Farbstoffs H in Schritt (9) erfolgt, eine photochrome Verbindung enthält.

**19.** Verfahren nach Anspruch 17 oder 18, **dadurch gekennzeichnet, daß** das 2-Propin-1-ol-derivat, mit dem die Umsetzung des Farbstoffs G in Schritt (8) erfolgt, eine photochrome Verbindung enthält.

**20.** Verfahren nach Anspruch 18 oder 19, **dadurch gekennzeichnet, daß** die photochrome Verbindung mehrere photochrome Farbzentren im Molekül besitzt.

**21.** Verwendung eines oder mehrerer der photochromen Naphthopyran-Farbstoffe nach einem der Ansprüche 1 bis 16 in Kunststoffen aller Art, insbesondere für ophthalmische Zwecke.

**22.** Verwendung eines oder mehrerer der photochromen Naphthopyran-Farbstoffe nach einem der Ansprüche 1 bis 16 in photochromen Gegenständen, insbesondere Linsen, Schutzgläsern, Visieren von Schutzhelmen, Fenstern, Brillengläsern, Schutzblenden, Abdeckungen, Dächern oder dergleichen.

**23.** Photochromer Gegenstand, umfassend ein oder mehrere photochrome Naphthopyran-Farbstoffe nach einem der Ansprüche 1 bis 16 sowie mindestens ein Polymermaterial.

**24.** Photochromer Gegenstand nach Anspruch 23, worin das Polymermaterial ausgewählt ist aus der Gruppe, bestehend aus Poly($C_1$-$C_{12}$-alkylmethacrylaten), Poly(oxyalkylendimethacrylaten), Poly(alkoxylierten Phenolmethacrylaten), Celluloseacetat, Cellulosetriacetat, Celluloseacetatpropionat, Celluloseacetatbutyrat, Poly(vinylacetat), Poly(vinylalkohol), Poly(vinylchlorid), Poly(vinylidenchlorid), thermoplastischen Polycarbonaten, Polyestern, Polyurethanen, Poly(ethylenterephthalat), Polystyrol, Poly($\alpha$-methylstyrol), Copoly(styrolmethylmethacrylat), Copoly (styrolacrylonitril), Polyvinylbutyral und Polymeren aus Bestandteilen der Gruppe, bestehend aus Polyol(allylcarbonat)monomeren, polyfunktionellen Acrylatmonomeren, polyfunktionellen Methacrylatmonomeren, Diethylenglycoldimethacrylatmonomeren, ethoxylierten Bisphenol-Adimethacrylatmonomeren, Diisopropenylbenzolmonomeren, Ethylenglycolbismethacrylatmonomeren, Poly(ethylenglycol)bismethacrylatmonomeren, ethoxylierten Phenolmethacrylatmonomeren, alkoxylierten Polyalkoholacrylatmonomeren und Diallylidenpentaerythritolmonomeren.

**25.** Photochromer Gegenstand nach Anspruch 23, worin das Polymermaterial ein festes oder transparentes Homo- oder Copolymer ist, ausgewählt aus der Gruppe, bestehend aus Poly(methylmethacrylat), Poly(ethylenglycolbismethacrylat), poly(ethoxyliertem Bisphenol-A-dimethacrylat), thermoplastischem Polycarbonat, Poly(vinylacetat), Polyvinylbutyral, Polyurethan oder ein Polymer, ausgewählt aus den Bestandteilen der Gruppe, bestehend aus Diethylenglycolbis(allylcarbonat)monomeren, Diethylenglycoldimethacrylatmonomeren, ethoxylierten Phenolmethacrylatmonomeren, Diisopropenylbenzolmonomeren und ethoxylierten Trimethylolpropantriacrylatmonomeren.

**26.** Photochromer Gegenstand nach einem der Ansprüche 23 bis 25, umfassend mindestens ein Polymermaterial nach Anspruch 24 oder 25 und ein oder mehrere photochrome Naphthopyran-Farbstoffe nach einem der Ansprüche 1 bis 16 sowie mindestens einen anderen organischen photochromen Farbstoff mit zumindest einem aktivierten Absorptionsmaximum im Bereich von etwa 400 bis 700 nm.

**27.** Photochromer Gegenstand nach einem der Ansprüche 23 bis 26, worin der oder die photochromen Naphthopyran-Farbstoffe in einer Menge von etwa 0,05 bis 2,5 mg pro $cm^2$ Polymermaterialoberfläche, in welche der oder die photochromen Farbstoffe eingebettet oder auf die sie aufgebracht sind, vorliegen.

**28.** Photochromer Gegenstand nach einem der Ansprüche 23 bis 27, erhältlich durch Massefärbung oder Oberflächenfärbung.

## Claims

**1.** Photochromic naphthopyran dyes having the general structural formula (I)

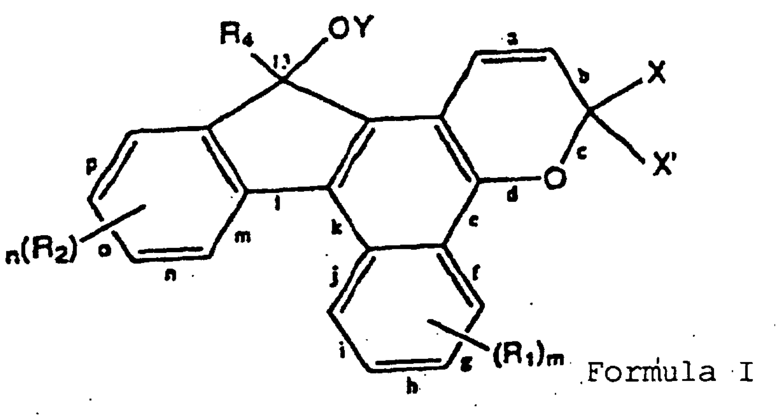

Formula I

wherein
$R_1$ and $R_2$, independently of one another, are identical or different and selected from
the group f, comprising ($C_1$-$C_6$)-alkyl, ($C_1$-$C_6$)-alkoxy, chloro and fluoro;
or
the group comprising hydrogen, ($C_5$-$C_6$)-cycloalkyl, phenyl, benzyl, dialkyl-($C_1$-$C_6$)-amino, dicyclohexylamino, diphenylamino, piperidyl, morpholinyl and pyridyl, wherein m, n = 0, 1 or 2;
or
two $R_1$ and/or two $R_2$ form together, independently of each other, a carbocyclic or heterocyclic ring, selected from

benzene, pyridine, pyrazine, pyrimidine, furan and thiophene;
$R_4$ is selected from
the group g, comprising ($C_5$-$C_6$)-cycloalkyl, ($C_1$-$C_6$)-acyl, ($C_1$-$C_6$)-alkoxy, phenyl, benzyl, mono-substituted phenyl, mono-substituted benzyl, the aryl substituents being ($C_1$-$C_6$)-alkyl or ($C_1$-$C_6$) alkoxy;
or from
monochloro- or monofluoro-substituted phenyl, naphthyl, phenanthryl, pyrenyl, quinolyl, isoquinolyl, benzofuranyl, benzothienyl, dibenzofuranyl, dibenzothienyl, carbazolyl, or indolyl, in each case unsubstituted or monosubstituted, the substituents being selected from the group comprising
chloro, fluoro
($C_1$-$C_6$)-alkyl or ($C_1$-$C_6$)-alkoxy;
or from phenyl di-or trisubstituted with substituents from the group comprising
chloro, fluoro, ($C_1$-$C_6$)-alkyl, ($C_1$-$C_6$)-alkoxy ($C_1$-$C_6$)-ω-phenylaklyl and ($C_1$-$C_6$)-ω-phenoxyaklyl, wherein the phenyl ring constantly at the -ω-position can here in turn represent the X, X' or $R_4$ residue of a further photochromic pyran system;
X and X' are selected independently of each other from the group z, comprising a) and b), with

a) being the un-, mono-, di- and trisubstituted aryl groups phenyl and naphthyl, and
b) the un-, mono-, di- and trisubstituted heterocyclic groups pyridyl, furanyl, benzofuran-2-yl, benzofuran-3-yl, thienyl, benzothien-2-yl and benzothien-3-yl,
and the substituents in a) and b) are selected from
the group h, comprising hydroxy, amino, mono-($C_1$-$C_6$)-alkylamino, di-($C_1$-$C_6$)-alkylamino, piperidino, morpholino, pyrryl, ($C_1$-$C_6$)-alkyl, ($C_1$-$C_6$)-chloroalkyl, ($C_1$-$C_6$)-fluoroalkyl, ($C_1$-$C_6$)-alkoxy, mono-($C_1$-$C_6$)-alkoxy-($C_1$-$C_4$)-alkyl, chloro and fluoro; or
the group a, comprising non-basic amines, such as unsubstituted or substituted diarylamino, pyrazole, imidazole, indole, pyridine, pyrazoline, imidazoline, pyrroline, phenothiazine, phenoxazine, phenazine, acridine or carbazole, the substituents on the non-basic amines being selected from ($C_{1-}C_4$)-alkyl, ($C_{1-}C_4$)-alkoxy, phenyl, fluoro, chloro and bromo;
or X and X' are selected from
c) ($C_{1-}C_6$)-alkyl, ($C_{1-}C_6$)-chloroalkyl, ($C_{1-}C_6$)-fluoroalkyl, mono-($C_{1-}C_6$)-alkoxy-($C_{1-}$C4)-alkyl, ($C_{3-}$ $C_6$)-cycloalkyl, mono-($C_{1-}C_6$)-alkoxy-($C_{3-}C_6$)-cycloalkyl, chloro-($C_{3-}C_6$)-cycloalkyl and fluoro-($C_{3-}C_6$)-cycloalkyl;
or
X and X' form together with the carbon atom at the point of linkage with the pyran a saturated $C_5$-$C_{10}$ monocyclic, $C_{7-}C_{12}$ bicyclic or $C_7$-$C_{12}$ tricyclic hydrocarbon ring or a fluorene-9-ylidene;
or
either X or X' is mono- or di-substituted phenyl which is substituted in the para-position for linking with -phenyl, $-(CH_2)_p$-phenyl or -O-$(CH_2)_p$-O-phenyl (p = 1, 2, 3 ..., 6), the second phenyl ring belonging to a second - not necessarily identical - photochromic pyran;

and
Y
is selected from the group comprising hydrogen, ($C_1$-$C_6$)-alkyl, ($C_1$-$C_6$)-acyl, unsubstituted benzoyl, unsubstituted naphthoyl, mono-substituted benzoyl and mono-substituted naphthoyl, the aryl substituents being ($C_1$-$C_6$)-alkyl or ($C_{1-}C_6$)-alkoxy;
with the proviso that $R_1$ and $R_2$ are not from the group f if $R_4$ is from the group g and X and X' are from the group z in the case where the residues of group z are unsubstituted or the substituents are from the group h.

2. Photochromic naphthopyran dyes according to claim 1, **characterised in that** the heterocycles in the group $R_4$ are connected to the naphthopyran system via the carbocyclic ring.

3. Photochromic naphthopyran dyes according to claim 1 or 2, **characterised in that** $R_1$, $R_2$, $R_4$ and Y are as defined in claim 1, and X and X' are selected from the group z defined in claim 1,
with the proviso that $R_1$ and $R_2$ are not from the group f if $R_4$ is from the group g and X and X' are from the group z, in the case where the residues of the group z are unsubstituted or the substituents are from the group h.

4. Photochromic naphthopyran dyes according to one of claims 1, 2 or 3, **characterised in that** two $R_1$ and/or two $R_2$ independently of each other form a carbocyclic or heterocyclic ring, selected from benzene, pyridine, pyrazine, pyrimidine, furan and thiophene.

**5.** Photochromic naphthopyran dyes according to one of claims 1 to 4, **characterised in that** at least one X or X' represents a residue, defined above, of the group z, said residue being substituted at least with a non-basic amine from the group a.

**6.** Photochromic naphthopyran dyes according to claim 5, **characterised in that** X and X' represent identical residues of the group z with different substituents.

**7.** Photochromic naphthopyran dyes according to claim 5 or 6, **characterised in that** the substituents on X and X' are identical.

**8.** Photochromic naphthopyran dyes according to one of claims 1 to 3, **characterised in that** $R_4$ is selected from the groups comprising phenyl residues substituted at least in the ortho-position, 1- or 2-naphthyl, 9-phenanthryl, pyrenyl and heteroaromatics such as benzofuranyl, benzothienyl, quinolyl or isoquinolyl.

**9.** Photochromic naphthopyran dyes according to one of claims 1 to 8, **characterised in that** one or more methoxy groups are present in the 5-, 6-, 7-and/or 8- position of the naphthopyran ring.

**10.** Photochromic naphthopyran dyes according to one of claims 1 to 9, **characterised in that** the photochromic dye contains a plurality of photochromic colour centres in the molecule.

**11.** Photochromic naphthopyran dyes according to claim 10, **characterised in that** the additional colour centre absorbs in a shorter or longer wavelength range than the photochromic colour centre already present.

**12.** Photochromic naphthopyran dyes according to one of the preceding claims, **characterised in that** the properties of the dyes such as absorption, migration and rate of brightening can be deliberately adjusted by the selection of the substituents.

**13.** Photochromic naphthopyran dyes according to one of the preceding claims, **characterised in that** the molecular weight of the dye can be adjusted and/or multiplied by the selection of the substituents.

**14.** Photochromic naphthopyran dyes having the following chemical structural formula (II)

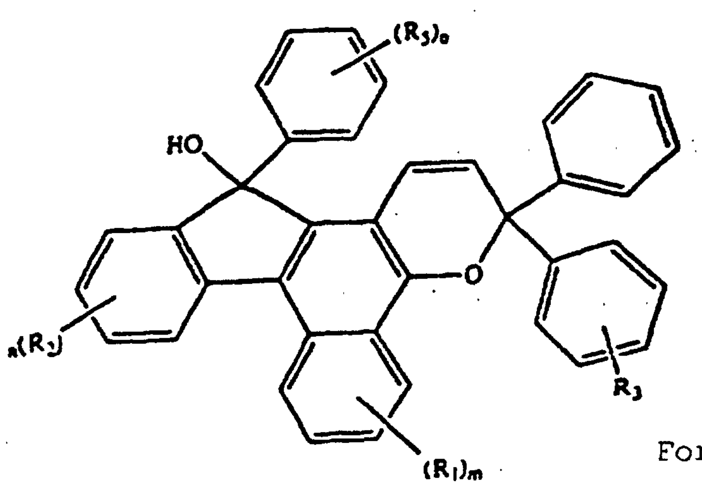

Formula (II)

wherein
$R_1$, $R_2$, m and n are as defined in claim 1,
$R_5$ represents the substituents defined for $R_4$ = phenyl, with o = 1, 2 or 3, and
$R_3$ is a non-basic amine from the group a defined in claim 1.

**15.** Photochromic naphthopyran dyes according to claim 14, **characterised in that** the non-basic amino substituent is unsubstituted or substituted diarylamino and at least one aryl ring can be mono-, di- or tri-substituted, the substituent or substituents, being selected from ($C_1$-$C_4$)-alkyl, ($C_1$-$C_4$)-alkoxy, phenyl, fluoro, chloro and bromo.

**16.** Photochromic naphthopyran dye selected from the group comprising

1) 6-methoxy-3-(4-methoxyphenyl)-3,13-diphenyl-13-hydroxybenz[p]indeno[2,1-f]naphtho[1,2-b]pyran,
2) 6-methoxy-3-(4-methoxyphenyl)-3-phenyl-13-(2,5-dimethylphenyl)-13-hydroxybenz[p]indeno[2,1-f]naphtho[1,2-b]pyran,
3) 3-(4-diphenylaminophenyl)-3-phenyl-13-(2,5-dimethylphenyl)-13-hydroxybenz[p]indeno[2,1-f]naphtho[1,2-b]pyran,
4) 3-(4-diphenylaminophenyl)-6-methoxy-3,13-diphenyl-13-hydroxyindeno[2,1-f]naphtho[1,2-b]pyran,
5) 3-(4-diphenylaminophenyl)-6-methoxy-3-phenyl-13-(2,5-dimethylphenyl)-13-hydroxy-indeno[2,1-f]naphtho[1,2-b]pyran,
6) 6-methoxy-3-(4-methoxyphenyl)-3-phenyl-13-(2,5-dimethylphenyl)-13-hydroxyindeno[2,1-f]naphtho[1,2-b]pyran,
7) 6-methoxy-3-(4-methoxyphenyl)-3-phenyl-13-(1-naphthyl)-13-hydroxyindeno[2,1-f]naphtho[1,2-b]pyran,
8) 6,11-dimethoxy-3-(4-methoxyphenyl)-3-phenyl-13-(2,5-dimethylphenyl)-13-hydroxyindeno[2,1-f]naphtho[1,2-b]pyran,
9) 3-(4-methoxyphenyl)-3-phenyl-13-(2,5-dimethylphenyl)-13-hydroxyindeno[2,1-f]naphtho[1,2-b]pyran, and
10) 6-methoxy-3-(4-methoxyphenyl)-3-phenyl-13-(3-methyl-2-thienyl)-13-hydroxyindeno[2,1-f]naphtho[1,2-b]pyran.

**17.** Method of producing the photochromic naphthopyran dyes according to one of claims 1 to 16, **characterised by** the following method steps (1) to (10) :

(1) reacting substituted or unsubstituted benzene or naphthalene compounds by Friedel Crafts acylation to form benzo- or naphthophenones A;

(2) carrying out a Stobbe condensation of compound A by means of succinodiesters to form an isomer mixture of semi-esters B;

(3) saponifying the semi-esters B with aqueous alkali to form the isomer mixture of diacids C;

(4) heating the diacids C with acetyl chloride with separation of water to form the isomer mixture of the cyclic anhydrides D;

(5) reacting the anhydrides D with aluminium chloride with intramolecular 5-ring cyclisation to form the'isomer mixture E and separating the isomers by means of solubility fractionation in benzene;

(6) reacting the corresponding isomer with acetic anhydride with intramolecular 6-ring cyclisation to form the ester F;

(7) subjecting the ester F to alkaline saponification to form the hydroxyfluorenone derivative G;

(8) reacting the compound G with a 2-propyn-1-ol derivative to form the photochromic indeno-annellated naphthopyran dye ,H;

(9) subjecting the dye H to a Grignard reaction with the compound $R_4MgHal$ to form the compound J, $R_4$ having the meaning given above and Hal being a halide ion; and

(10) derivatisation of the free hydroxyl group of compound J to form compound K.

**18.** Method according to claim 17, **characterised in that** the Grignard reagent used to react dye H in step (9) contains a photochromic compound.

**19.** Method according to claim 17 or 18, **characterised in that** the 2-propyn-1-ol derivative used to react dye G in step (8) contains a photochromic compound.

**20.** Method according to claim 18 or 19, **characterised in that** the photochromic compound has a plurality of photochromic colour centres in the molecule.

**21.** Use of one or more of the photochromic naphthopyran dyes according to one of claims 1 to 16 in plastics materials of all types, especially for ophthalmic purposes.

**22.** Use of one or more of the photochromic naphthopyran dyes according to one of claims 1 to 16 in photochromic objects, especially lenses, protective glasses, visors of protective helmets, windows, spectacle glasses, protective screens, covers, roofs or the like.

**23.** Photochromic object comprising one or more photochromic naphthopyran dyes according to one of claims 1 to 16 as well as at least one polymer material.

**24.** Photochromic object according to claim 23, wherein the polymer material is selected from the group comprising poly($C_1$-$C_{12}$-alkyl methacrylates), poly(oxalkylene dimethacrylates), poly(alkoxylated phenol methacrylates), cellulose acetate, cellulose triacetate, cellulose acetate propionate, cellulose acetate butyrate, poly(vinyl acetate), poly(vinyl alcohol) poly(vinyl chloride), poly(vinylidene chloride), thermoplastic polycarbonates, polyesters, polyurethanes, poly(ethylene terephthalate) polystyrene, poly($\alpha$-methyl styrene), copoly-(styrene methyl methacrylate), copoly(styrene acrylonitrile), polyvinyl butyral, and polymers from members of the group comprising polyol (allylcarbonate)monomers, polyfunctional acrylate monomers, polyfunctional methacrylate monomers, diethylene glycol dimethacrylate monomers, ethoxylated bisphenol-A-dimethacrylate monomers, diisopropenyl benzene monomers, ethylene glycol bismethacrylate monomers, poly(ethylene glycol)bismethacrylate monomers, ethoxylated phenol methacrylate monomers, alkoxylated polyalcohol acrylate monomers and diallylidene pentaerythritol monomers.

**25.** Photochromic object according to claim 23 wherein the polymer material is a solid or transparent homo- or copolymer, selected from the group comprising poly(methyl methacrylate), poly(ethylene glycol bismethacrylate), poly(ethoxylated bis-phenol-A-dimethacrylate), thermoplastic poly-carbonate, poly(vinyl acetate), polyvinylbutyral, polyurethane or a polymer, selected from the members of the group comprising diethylene glycol bis(allyl carbonate) monomers, diethylene glycol dimethacrylate monomers, ethoxylated phenol methacrylate monomers, diisopropenyl benzene monomers and ethoxylated trimethylol propane triacrylate monomers.

**26.** Photochromic object according to one of claims 23 to 25, comprising at least one polymer material according to claim 24 or 25 and one or more photochromic naphthopyran dyes according to one of claims 1 to 16 as well as at least one other organic photochromic dye having at least an activated absorption maximum in the range from about 400 to 700 nm.

**27.** Photochromic object according to one of claims 23 to 26, wherein the photochromic naphthopyran dye or dyes are present in an amount of roughly 0.05 to 2.5 mg per $cm^2$ of the surface of the polymer material in which the photochromic dye or dyes are embedded and to which they are applied.

**28.** Photochromic object according to one of claims 23 to 27, which can be obtained by colouring of the mass or by surface colouring.

## Revendications

**1.** Colorants naphthopyranes photochromiques présentant une formule de structure générale

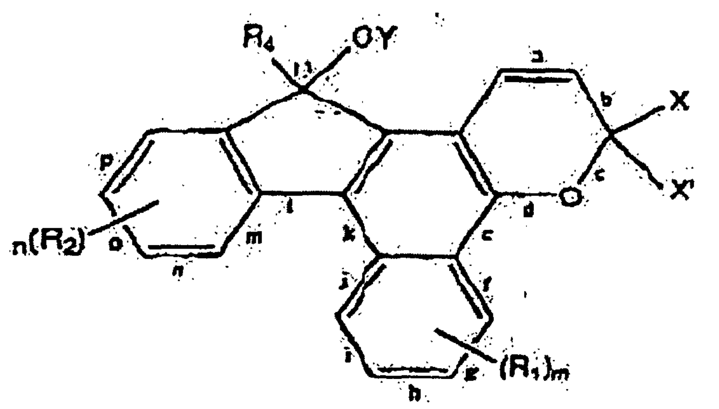

Formule (1)

dans laquelle
$R_1$ et $R_2$
sont indépendamment l'un de l'autre différents ou similaires et sont sélectionnés à partir du groupe f, se composant d'alkyle en ($C_1$-$C_6$), d'alcoxy en ($C_1$-$C_6$), de chloro et de fluoro ;
ou
dans le groupe, se composant d'hydrogène, de cycloalkyle en ($C_5$-$C_6$), de phényle, de benzyle, de dialkyl-($C_1$-$C_6$)-amino, de dicyclohexylamino, de diphénylamino, de pipéridyle, de morpholinyle et de pyridyle, où m, n = 0,1 ou 2 ;
ou
deux $R_1$ et/ou deux $R_2$ forment ensemble ou indépendamment l'un de l'autre un anneau hétérocyclique ou carbocyclique sélectionné à partir du benzène, de la pyridine, de la pyrazine, de la pyrimidine, du furane et du thiophène ;
$R_4$ est sélectionné à partir du groupe G, se composant de cycloalkyle en ($C_5$-$C_6$), d'acyle en ($C_1$-$C_6$), d'alcoxy en ($C_1$-$C_6$), de phényle, de benzyle, de phényle monosubstitué, de benzyle monosubstitué, où les substituts aryles sont de l'alkyle en ($C_1$-$C_6$) ou de l'alcoxy en ($C_1$-$C_6$) ;
ou
à partir monochloro substitué ou monofluoro substitué de phényle, de naphthyle, de phénanthryle, de pyrényle, de quinolyle, d'isoquinolyle, de benzofuranyle, de benzothiényle, de dibenzofuranyle, de dibenzothiényle, de carbazolyle, ou d'indolyle, monosubstitué ou non-substitué à chaque fois, où les substituants sont sélectionnés à partir du groupe se composant de chloro, fluoro, d'alkyle en ($C_1$-$C_6$) ou d'alcoxy en ($C_1$-$C_6$) ;
ou
qui sont sélectionnés à partir de phényle di- ou tri-substitué provenant des groupes se composant du chloro, du fluoro, de l'alkyle en ($C_1$-$C_6$), de l'alcoxy en ($C_1$-$C_6$), ($C_1$-$C_6$)-ω-phénylalkyle et ($C_1$-$C_6$)- ω-phénoxyalkyle, ou le cycle phényle ω constant peut représenter tour à tour le X, X' ou les restes $R_4$ d'un autre système pyrane photochromique ;
X et X'
sont sélectionnés indépendamment l'un de l'autre à partir du groupe z, se composant de a) et b) comprenant

a) les groupes non-substitués, monosubstitués, di- ou tri-substitués de phényle et de naphthyle et
b) les groupes hétérocycliques non-substitués, monosubstitués, di- ou tri-substitués pyridile, furanyle, benzofuran-2-yle, benzofuran-3-yle, thiényle, benzothièn-2-yle et benzothièn-3-yle,
et les substituants dans a) et b)
sont sélectionnés à partir du groupe h, se composant d'hydroxy, d'amino, de mono-($C_1$-$C_6$)-alkylamino, de di-($C_1$-$C_6$)-alkylamino, de pipéridino, de morpholino, de pyrryle, de ($C_1$-$C_6$)-alkyle, de ($C_1$-$C_6$)-chloroalkyle, de ($C_1$-$C_6$)-fluoroalkyle, de ($C_1$-$C_6$)-alcoxy, de mono- ($C_1$-$C_6$)-alcoxy-($C_1$-$C_4$)-alkyle, chloro et fluoro ; ou
le groupe a, se composant d'amines non basiques, comme des diarylamino substitués ou non-substitués, du pyrazole, de l'imidazole, de l'indole, de la pyridine, de la pyrazoline, de l'imidazoline, de la pyrroline, de la phénothiazine, de la phénoxazine, de la phénazine, de l'acridine ou du carbazole, où les substitués au niveau des amines non basiques sont sélectionnés à partir d'alkyle en ($C_1$-$C_4$), d'alcoxy en ($C_1$-$C_4$), de phényle, de fluoro, de chloro et de bromo ;
ou sont sélectionnés à partir de
c) d'alkyle en ($C_1$-$C_6$), de chloroalkyle en ($C_1$-$C_6$), de fluoroalkyle en ($C_1$-$C_6$), de mono-($C_1$-$C_6$)-alcoxy-($C_1$-C4)-alkyle, de ($C_3$-$C_6$)-cycloalkyle, de mono-($C_1$-$C_6$)-alcoxy-($C_3$-$C_6$)-cycloalkyle, de chloro-($C_3$-$C_6$)-cycloalkyle et

de fluor-($C_3$-C6)-cycloalkyle ;

ou

X et X' forment avec l'atome de carbone du site de liaison avec le pyrane un cycle de carbone saturé $C_5$-$C_{10}$ monocyclique, $C_7$-$C_{12}$ bicyclique ou $C_7$-$C_{12}$ tricyclique ou un fluorèn-9-ylidène ;

ou

soit X soit X' est un phényle monosubstitué ou disubstitué, qui est substitué sur le site para de la liaison avec le -phényle, avec le -$(CH_2)_p$-phényle ou -O-$(Ch_2)_p$-O-phényle ou et substitué sur le site para pour la liaison avec le -phényle -(p = 1,2,3....,6), ou le deuxième cycle phényle appartient à un deuxième - qui n'est pas nécessairement identique au pyrane photochromique ;

et Y

sélectionné à partir du groupe se composant d'hydrogène, d'alkyle en ($C_1$-$C_6$), d'acyle en ($C_1$-$C_6$), de benzoyle non-substitué, de naphthoyle non-substitué, de benzoyle monosubstitué et de naphthoyle monosubstitué , où les substituants d'aryle sont des alkyles en ($C_1$-$C_6$) ou des alcoxy en ($C_1$-$C_6$) ;

à condition que $R_1$ et $R_2$ ne soient pas issus du groupe f, si $R_4$ provient du groupe g et X et X' proviennent du groupe z, au cas où les restes du' groupe z sont substitués ou non-substitués par le groupe h.

**2.** Colorants naphthopyranes photochromiques selon la revendication 1, **caractérisés en ce que** les hétérocycles dans le groupe $R_4$ sont liés via si un carbocycle avec un système naphthopyrane.

**3.** Colorants naphthopyranes photochromiques selon la revendication 1 ou 2, **caractérisés en ce que** $R_1$, $R_2$, $R_4$ et Y sont définis comme dans la revendication 1 et X et X' sont sélectionnés à partir du groupe z tel que défini dans la revendication 1, à condition que $R_1$ et $R_2$ ne soient pas issus du groupe f, quand $R_4$ provient du groupe g et que X et X' proviennent du groupe z si les restes du groupe z sont non-substitués ou sont des substituants sélectionnés dans le groupe h.

**4.** Colorants naphthopyranes photochromiques selon l'une quelconque des revendications 1, 2 ou 3, **caractérisés en ce que**, deux $R_1$ et/ou deux $R_2$, forment indépendamment l'un de l'autre un anneau hétérocyclique ou carbocyclique sélectionné parmi le benzène, la pyridine, la pyrazine, la pyrimidine, le furanne et le thiophène.

**5.** Colorants naphthopyranes photochromiques selon l'une quelconque des revendications 1 à 4, **caractérisés en ce qu'**au moins un X ou X' représente au moins avec une amine non-basique issue du groupe a, un reste substitué défini précédemment provenant du groupe z.

**6.** Colorants naphthopyranes photochromiques selon la revendication 5, **caractérisés en ce que** X et X' représentent les mêmes restes choisis dans le groupe z avec des substituants différents.

**7.** Colorants naphthopyranes photochromiques selon la revendication 5 ou 6, **caractérisés en ce que** les substituants au niveau de X et de X' sont identiques.

**8.** Colorants naphthopyranes photochromiques selon l'une des revendications 1 à 3, **caractérisés en ce que** $R_4$ est sélectionné à partir du groupe se composant d'au moins des restes phényles substitués en ortho-position, du 1-ou 2-naphthyle, du 9-phénanthryle, du pyrénile ou des hétéroatomes, tels que le benzofuranyle, le benzothiényle, la quinolyle et l'isoquinolyle.

**9.** Colorants naphthopyranes photochromiques selon l'une quelconque des revendications 1 à 8, **caractérisés en ce que** un ou plusieurs groupes méthoxy existent en position 5, 6, 7 et/ou 8 du cycle naphthopyrane.

**10.** Colorants naphthopyranes photochromiques selon l'une quelconque des revendications 1 à 9, **caractérisés en ce que** le colorant photochromique contient plusieurs centres colorants photochromiques dans la molécule.

**11.** Colorants naphthopyranes photochromiques selon la revendication 10, **caractérisés en ce que** l'autre centre de colorant photochromique absorbe une plage de longueurs d'onde plus longue ou inférieure à celle du centre colorant photochromique existant déjà.

**12.** Colorants naphthopyranes photochromiques selon l'une quelconque des revendications précédentes, **caractérisés en ce que** la propriété des colorants comme l'absorption, la migration, la vitesse d'éclaircissement peuvent être définies de façon ciblée par le choix des substituants.

**13.** Colorants naphthopyranes photochromiques selon l'une quelconque des revendications précédentes, **caractérisés en ce que** le poids moléculaire du colorant peut être paramétré par le choix des substituants et / ou multiplié.

**14.** Colorants naphthopyranes photochromiques présentant la formule de structure chimique suivante (II) :

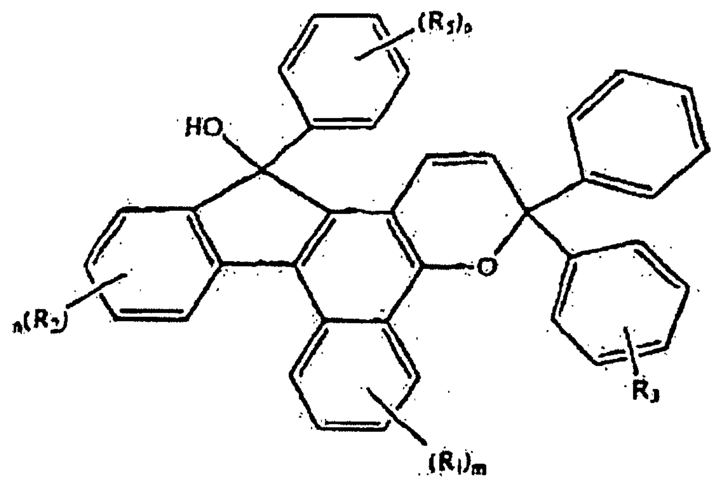

Formule (II)

où
$R_1$, $R_2$ m et n sont tels que définis dans la revendication 1
$R_5$ représente $R_4$ = des substituants définis en phényle avec 0 = 1, 2 ou 3 et
$R_3$ est une amine, non-basique provenant du groupe a, définie dans la revendication 1.

**15.** Colorants naphthopyranes photochromiques selon la revendication 14, **caractérisés en ce que** l'amino substituant non-basique est un diarylamino non-substitué ou substitué et qu'au moins un cycle aryle puisse être mono-, di- ou tri-substitué ou le ou les substituants sont sélectionnés à partir de l'alkyle en ($C_1$-$C_4$), de l'alcoxy en ($C_1$-$C_4$), du phényle, du fluoro, du chloro et du bromo.

**16.** Colorants naphthopyranes photochromiques sélectionnés à partir du groupe se composant de

1) 6-méthoxy-3-(4-méthoxyphényl)-3,13-diphényl-13-hydroxy-benz[p]indeno[2,1-f]naphtho[1,2-b]pyrane,
2) 6-méthoxy-3-(4-méthoxyphényl)-3-phényl-13-(2,5-diméthylphényl)-13-hydroxybenz[p]indeno[1,2-f]naphtho[1,2-b]pyrane,
3) 3-(4-diphénilaminophényl)-3-phényl-13-(2,5-diméthylphényl)-13-hydroxy-benz[p]indeno[2,1-f]naphtho[1,2-b]pyrane,
4) 3-(4-diphénilaminophényl)-6-méthoxy-3,13-diphényl-13-hydroxy-indeno[2,1-f]naphtho[1,2-b]pyrane,
5) 3-(4-diphénylaminophényl)-6-méthoxy-3-phényl-13-(2,5-diméthylphényl)-13-hydroxy-indeno[2,1-f]naphtho[1,2-b]pyrane,
6) 6-méthoxy-3-(4-méthoxyphényl)-3-phényl-13-(2,5-diméthylphényl)-13-hydroxyindeno[2,1-f]naphtho[1,2-b]pyrane,
7) 6-méthoxy-3-(4-méthoxyphényl)-3-phényl-13-(1-naphthyl)-13-hydroxy-indeno[2,1-f]naphto[1,2-b]pyrane,
8) 6,11-diméthoxy-3-(4-méthoxyphényl)-3-phényl-13-(2,5-diméthyl-phényl)-13-hydroxy-indeno[2,1-f]naphtho[1,2-b]pyrane,
9) 3-(4-méthoxyphényl)-3-phényl-13-(2,5-diméthylphényl)-13-hydroxy-indeno[2,1-f]naphtho[1,2-b]pyrane et
10) 6-méthoxy-3-(4-méthoxyphényl)-3-phényl-13-(3-méthyl-2-thiényl)-13-hydroxyindeno[2,1-f]naphtho[1,2-b]pyrane.

**17.** Procédé pour la fabrication d'un colorant naphthopyrane photochromique selon l'une quelconque des revendications 1 à 16, **caractérisé par** les étapes de processus suivants de (1) à (10) :

(1) Conversion des composés substitués ou non-substitués naphthaline et benzène par acylation Friedel-Crafts en benzo-phénone ou en naphtophénone A ;
(2) Réalisation d'une condensation de Stobbe du composé A au moyen de diester d'acide succinique en un mélange isomère de demi-ester B ;
(3) Saponification du demi-ester B avec un alcalin aqueux en un mélange isomère de diacide C ;
(4) Chauffage du diacide C à l'aide de chlorure d'acide sous séparation aqueuse en un mélange isomère d'anhydride cyclique D ;
(5) Conversion de l'anhydride D à l'aide de chlorure d'aluminium sous cyclisation intramoléculaire à 5 cycles en un mélange isomère E et séparation de l'isomère au moyen d'un fractionnement de solubilité dans du benzène ;
(6) Conversion de l'isomère correspondant à l'aide d'anhydride d'acétate sous cyclisation intramoléculaire à 6 cycles en ester F ;
(7) Saponification alcaline de l'ester F en un dérivé G hydroxy-fluorénone ;
(8) Conversion du composé G à l'aide d'un dérivé 2-propin-1-ol pour un colorant naphthopyrane indenoannelé photochromique H ;
(9) Conversion Grignard du colorant H avec le composé $R_4$MgHal en un composé J, ou $R_4$ possède la signification sus-citée et Hal est un ion halogène ; et
(10) Dérivation des groupes hydroxy libres du composé J en composé K.

**18.** Procédé selon la revendication 17, **caractérisé en ce que** le réactif Grignard avec lequel la conversion du colorant H lors de l'étape (9) est réalisée, contient un composé photochromique.

**19.** Procédé selon la revendication 17 ou 18, **caractérisé en ce que** le dérivé 2-propin-1-ol, avec lequel est réalisée la conversion du colorant G lors de l'étape (8), contient un composé photochromique.

**20.** Procédé selon la revendication 18 ou 19, **caractérisé en ce que** le composé photochromique possède plusieurs centres colorants photochromiques dans la molécule.

**21.** Utilisation d'un ou plusieurs colorants naphthopyranes photochromiques selon l'une quelconque des revendications 1 à 16 dans du plastique de toutes sortes notamment pour des desseins ophtalmiques.

**22.** Utilisation d'un ou plusieurs colorants naphthopyranes photochromiques selon l'une des revendications 1 à 16 dans des objets photochromiques, en particulier des lentilles, des verres de protection, des verres de masque de protection, des fenêtres, des verres pour lunettes, des diaphragmes de protection, des couvertures, des toits ou similaires.

**23.** Objet photochromique comprenant un ou plusieurs colorants naphthopyranes photochromiques selon l'une quelconque des revendications 1 à 16 ainsi qu'au moins un matériau polymère.

**24.** Objet photochromique selon la revendication 23, dans lequel le matériau polymère est sélectionné à partir du groupe se composant de Poly($C_1$-$C_{12}$-alkyl-méthacrylate), poly(oxyalkylendiméthacrylate), poly(alcoxylé phénolméthacrylate), d'actétate de cellulose, de triacétates de cellulose, de propionate d'acétate de cellulose, de butirate d'acétate de cellulose, de poly(vinylacétate), poly(vynilalcool), chlorure de poly(vinylique), chlorure de polyvinylidène, polycarbonate thermoplastique, polyesters, polyuréthannes, poly(éthylentérephtalate), polystyrole, poly($\alpha$-méthylstyrole), copoly(styrolméthylméthacrylate), copoly(styrolacrylonitrile), polyvinylbutyral et polymères se composant d'éléments du groupe consistant en des monomères polyol(allylcarbonate), des monomères d'acrilate polyfonctionnels, des monomères de méthacrylate polyfonctionnels, des monomères de diéthylglycoldiméthacrylate, des monomères bisphénol-A-diméthacrylate, des monomères de diisopropénylbenzène, des monomères d'éthylènglycolbisméthacrylate, des monomères de poly(éthylènglycol)bisméthacrylate, des monomères de phénolméthacrylate éthoxylé, des monomères de polyalcool d'acrylate alcoxylés et des monomères de diallylidène pentaérythritol.

**25.** Objet photochromique selon la revendication 23, dans lequel le matériau polymère est un copolymère ou un homopolymère transparent ou solide, sélectionné à partir du groupe se composant du poly(méthylméthacrylate), de poly(éthylène glycol-bisméthacrylate), de poly(éthoxylé bisphénol-A-diméthacrylate), de polycarbonate thermoplastique, d'acétate polyvinylique, de polyvinylbutyral, de polyuréthanne ou d'un polymère, se composant des éléments du groupe consistant en de monomère diéthylène glycol-bis(allylcarbonate), du monomère de dyéthylène glycol-diméthacrylate, du monomère de phénolméthacrylate éthoxylé, des monomères de diisopropénylbenzène et des monomères de triméthylpropantriacrylate.

**26.** Objet photochromique selon l'une quelconque des revendications 23 à 25, comprenant au moins un matériau polymère selon la revendication 24 ou 25 et un ou plusieurs colorants naphthopyranes photochromiques selon l'une quelconque des revendications 1 à 16 ainsi qu'au moins un autre colorant organique photochromique comprenant au moins un maximum d'absorption activée dans une plage allant de 400 à 700 nm.

**27.** Objet photochromique selon l'une quelconque des revendications 23 à 26, dans lequel le ou les colorants naphthopyranes photochromiques sont présents dans une quantité d'environ 0,05 à 2,5 mg par $cm^2$ de surface de matériaux polymères dans lequel le ou les colorants photochromiques sont intégrés ou sont appliqués à la surface de celui-ci.

**28.** Objet photochromique selon l'une quelconque des revendications 23 à 27, disponible à partir de coloration de masse ou de coloration en surface.

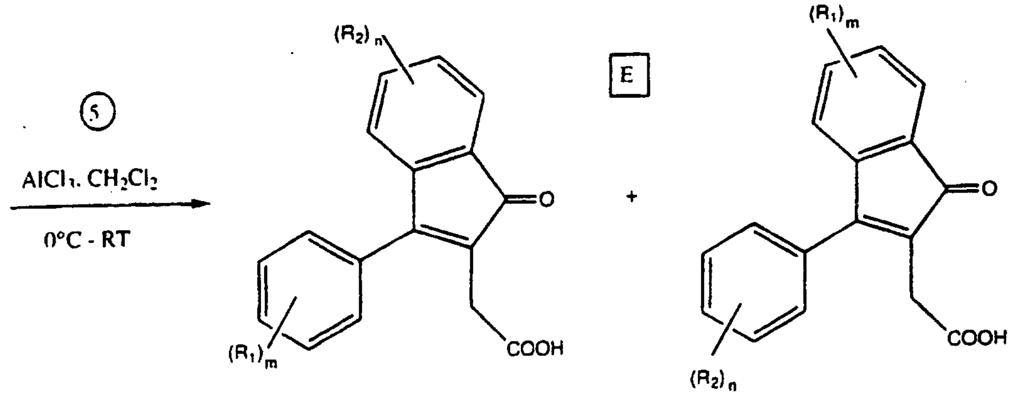


E

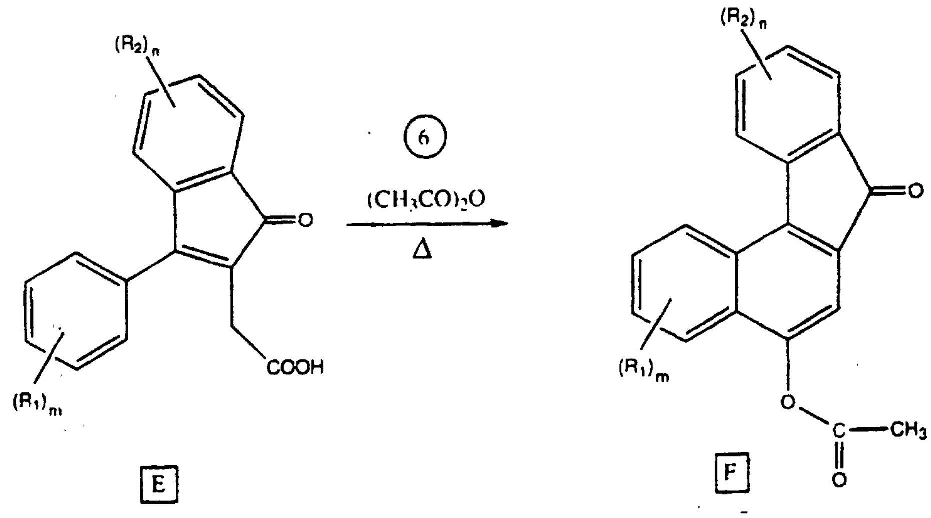


E

F

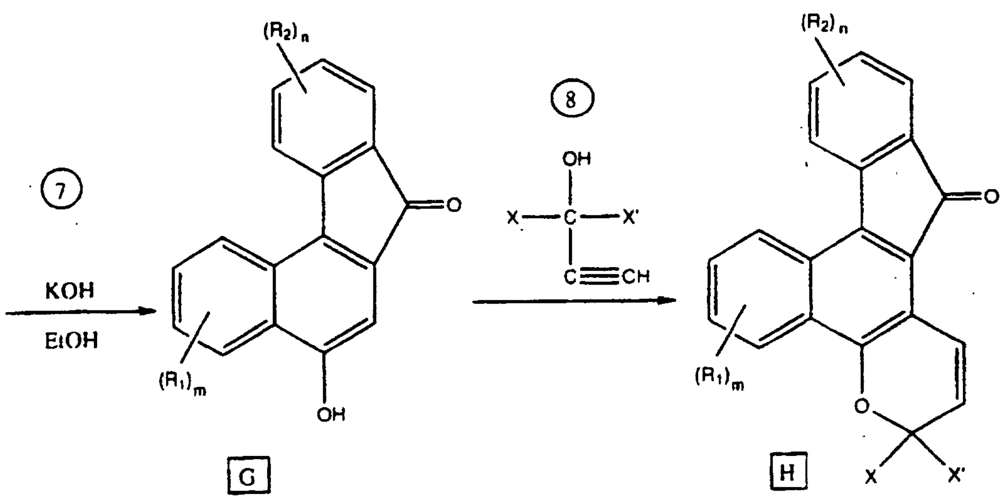


G

H

Fig. 1 Fortsetzung

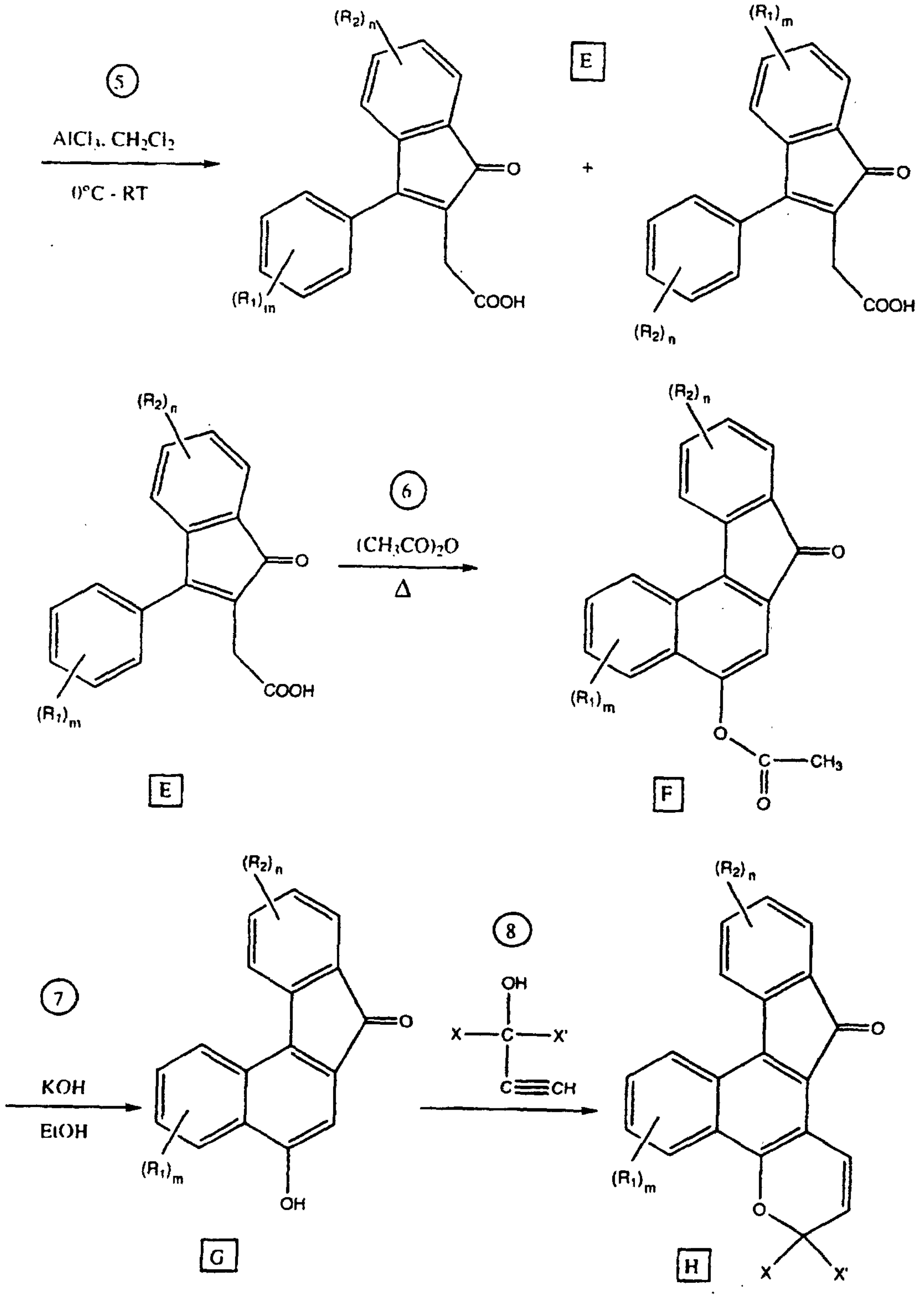


Fig. 1 Fortsetzung

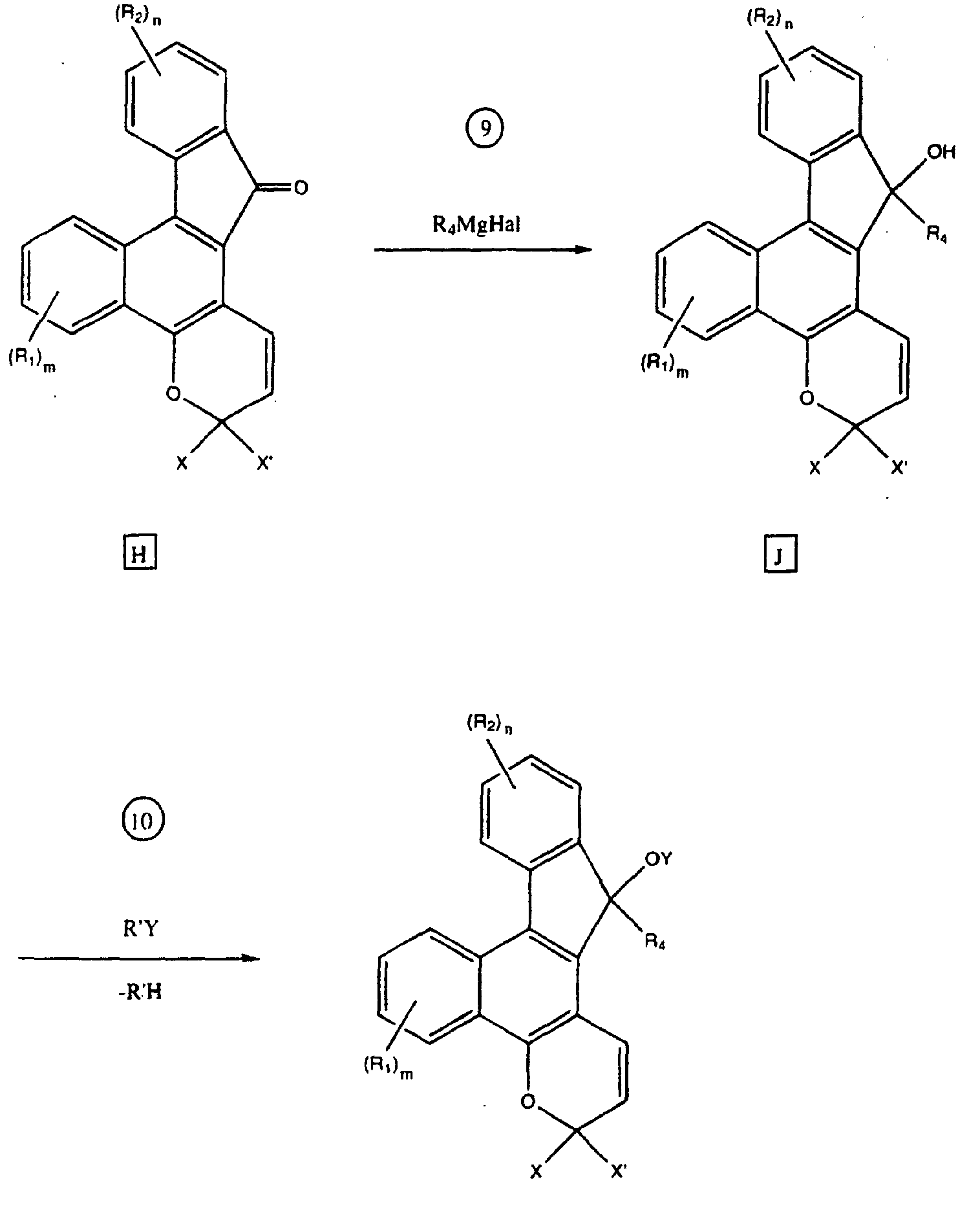


H

J

K

Fig. 2

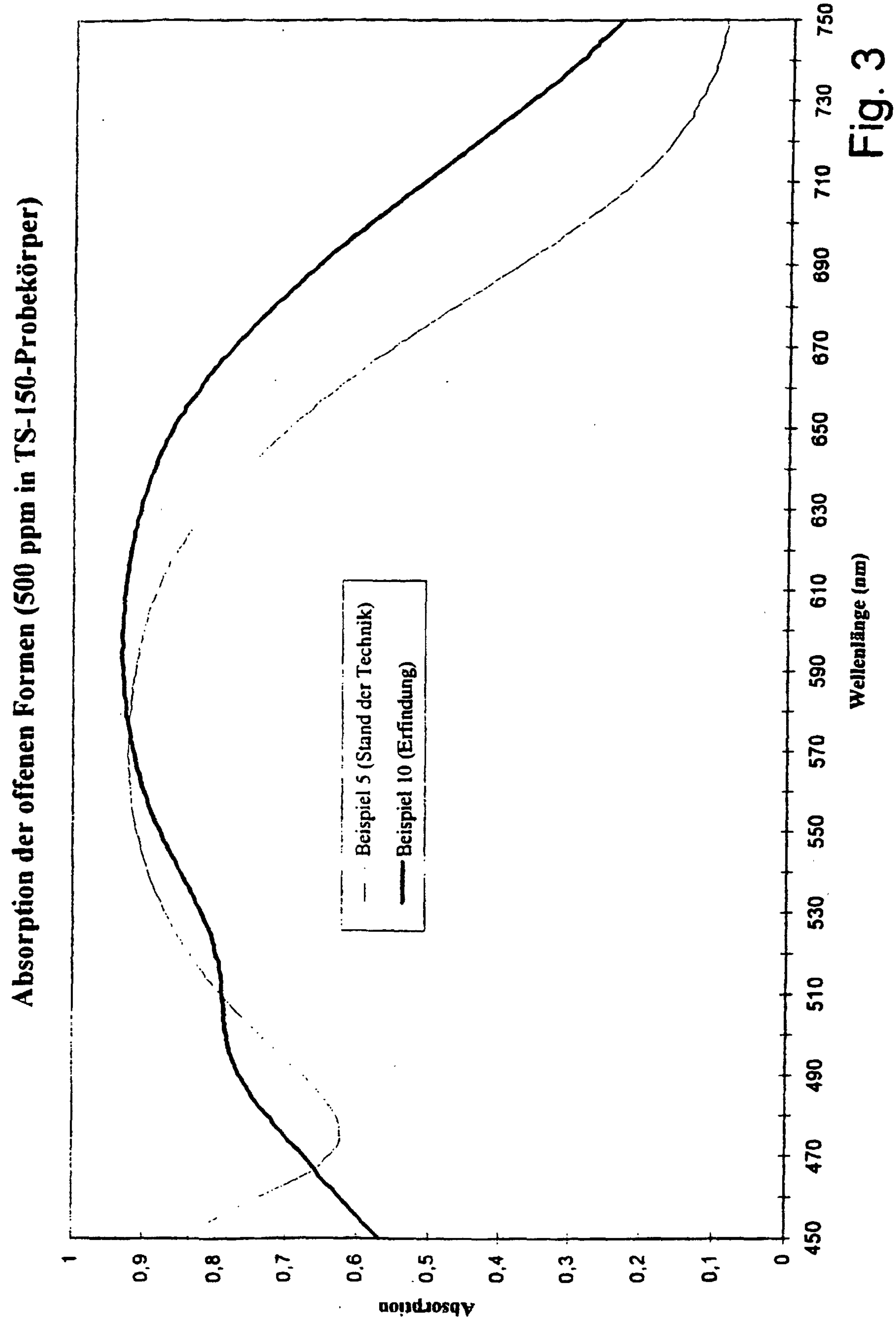
Absorption der offenen Formen (500 ppm in TS-150-Probekörper)
Beispiel 5 (Stand der Technik)
Beispiel 10 (Erfindung)
Absorption
1
0,9
0,8
0,7
0,6
0,5
0,4
0,3
0,2
0,1
0
450
470
490
510
530
550
570
590
610
630
650
670
690
710
730
750
Wellenlänge (nm)

Fig. 3

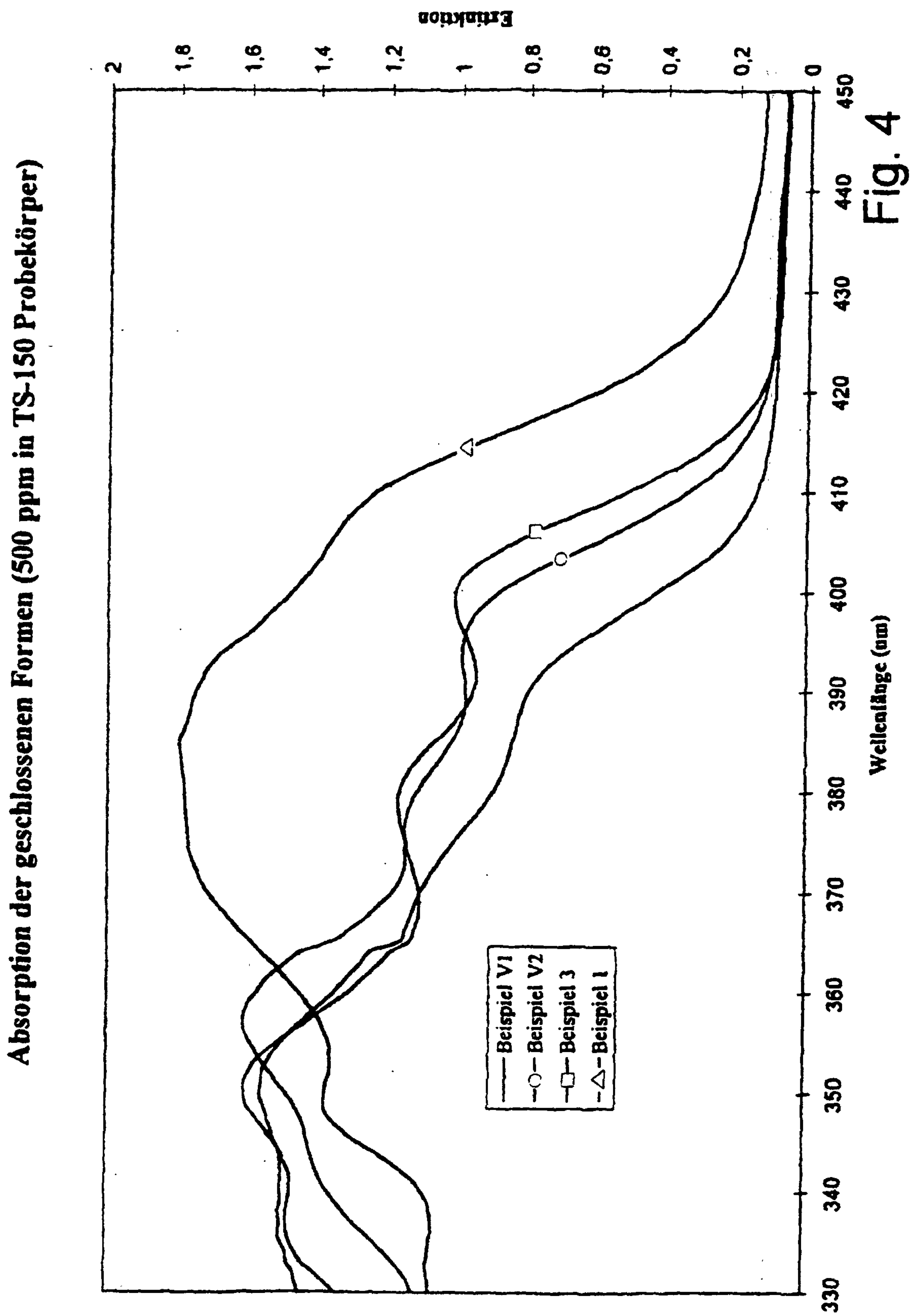

Absorption der geschlossenen Formen (500 ppm in TS-150 Probekörper)
Extinktion
2
1,8
1,6
1,4
1,2
1
0,8
0,6
0,4
0,2
0
330
340
350
360
370
380
390
400
410
420
430
440
450
Wellenlänge (nm)
Beispiel V1
Beispiel V2
Beispiel 3
Beispiel 1


Fig. 4

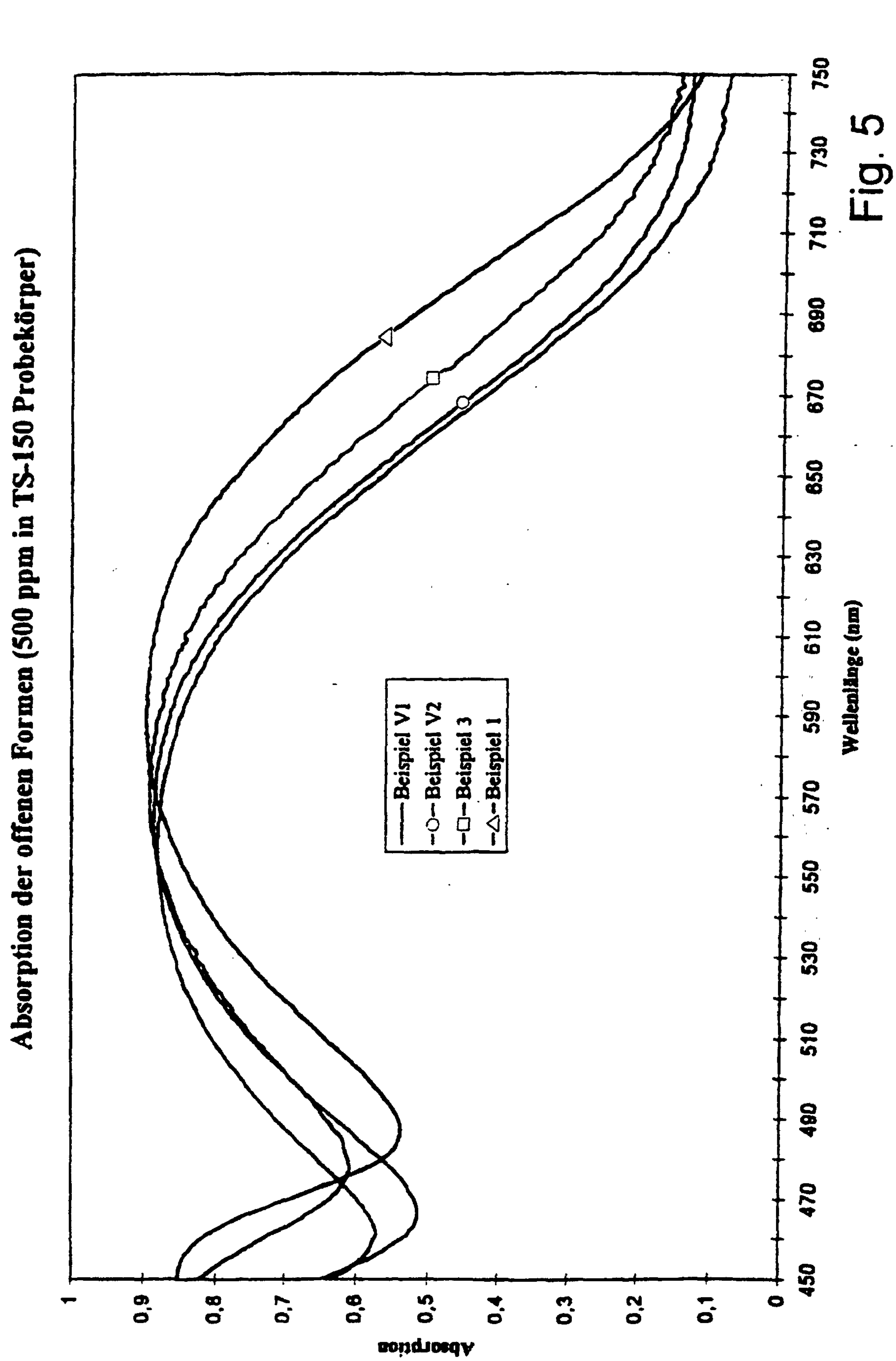
Absorption der offenen Formen (500 ppm in TS-150 Probekörper)
Absorption
1
0,9
0,8
0,7
0,6
0,5
0,4
0,3
0,2
0,1
0
450
470
490
510
530
550
570
590
610
630
650
670
690
710
730
750
Wellenlänge (nm)
Beispiel V1
Beispiel V2
Beispiel 3
Beispiel 1

Fig. 5